# EUROPEAN PATENT APPLICATION

(11) **EP 4 830 626 A2**
(43) Date of publication of application: **28.10.2026**
(21) Application number: 26204201.3
(22) Date of filing: 17.05.2023
(51) Int. Cl.: A61B 5/1455

(54) **OPTICAL SENSOR AND ASSOCIATED METHODS**

(30) Priority: 18.05.2022 EP 22174066; 18.05.2022 EP 22174067; 19.05.2022 EP 22174277; 04.01.2023 EP 23150330
(62) Document number(s) of the earlier application(s) in accordance with Art. 76 EPC: 23729311.3 / 4 525 714
(71) Applicant: Universität Zürich, 8006 Zürich (CH)
(72) Inventor: Kalyanov, Alexander, 5507 Mellingen (CH); Kleiser, Stefan, 79639 Grenzach-Wyhlen (DE); Nussli, Anais, 9500 Wil (CH); Ostojic, Daniel, 8046 Zurich (CH); Wolf, Martin, 8038 Zurich (CH)
(74) Representative: Maucher Jenkins Patent- und Rechtsanwälte Partnerschaft mbB

(57) **Abstract**

This document provides various technical improvements for performing optical NIRS measurements using an optical sensor. The revelation concerns A) a specific measurement method, B) a specific cable design for such a sensor, C) a light shielding cover to be used with such a sensor, and D) a specific internal light shielding to be used inside the sensor. All of these aspects can be used for improving the accuracy and robustness of the optical measurements to be performed with the sensor (cf. Fig. A1).

## Description

The present disclosure relates to the field of optical near infrared spectroscopy (NIRS) but can also be employed in other optical measurements. The present disclosure concerns an optical sensor, which can be configured as an optical NIRS sensor, and associated methods. All four aspects now detailed in the following may be used in combination, i.e. applied to one particular sensor, for improving the accuracy of optical measurements performed with such a sensor.

In one **aspect B)**, the present disclosure concerns an optical sensor and a method for fabricating the same using a molding step.

The sensor may be configured as a NIRS-sensor or as an oximeter, for example, such that it can be used for measuring a physiological parameter in human tissue. The sensor comprises a sensor head bearing at least one light source and at least one detector which are arranged on a carrier substrate. The sensor head may define a contact plane to be brought into skin contact during a measurement. Finally, the sensor features a cable comprising at least one signal carrier that is configured to route signals to and/or from the sensor head, for example to and/or from an external electronic or optical unit.

Such sensors are already used in clinical trials for measuring blood oxygenation in cerebral tissue of neonates. In such an application case, the sensor head must be brought in close vicinity to the curved shape of the head of the neonate. Any gap between a lower contact surface of the sensor head and the skull of the neonate must be avoided because this would allow ambient light to reach the measurement spot, which would deteriorate the measurement.

At the same time, the cable is necessary for a reliable measurement. The cable however, if formed too stiff, may get in conflict with the aim of attaching the sensor head in close skin contact, as the cable can limit the amount of bending that is achievable with the flexible sensor head.

Starting out from this background, the invention aims at providing a simple fabrication method and sensor design, which alleviates the afore-mentioned problem.

In accordance with the present invention, an optical sensor is provided according to claim 1, which solves the afore-mentioned problem. In particular, the invention proposes an optical sensor as introduced at the beginning, which, in addition, is characterized in that the carrier substrate is elevated above the contact plane by a distance D1.

Most preferably a middle portion of the at least one signal carrier, being located between the carrier substrate and a proximal portion of the at least one signal carrier, may follow a smooth curve. "Smooth" may be understood here, in particular, in that the curve shows no sharp kinks. Instead, the smooth curve shows a continuous course.

According to one possible embodiment, which is advantageous if the cable runs roughly parallel to the contact plane, the curve may show an S-shape. In other words, the middle portion may follow a smooth S-shaped curve.

Due to the smooth, in particular S-shaped, curve, the middle portion can thus bend downwards (along the z-axis) from the elevated level in which the carrier substrate is located to a lower level in which said contact plane is located. In other words, the at least one signal carrier may be designed as a flexible component, such that it offers no shape stability (other than for example a stiff PCB or a metal member).

As a major benefit of this approach, during an optical measurement, when the sensor head is attached onto the skull of a neonate, the portion of the signal carrier (which may be implemented as a flex-PCB for example) that extends into the cable can exit the head of the sensor as close to the patient's skin and as parallel thereto as possible. This avoids the formation of pressure marks on the delicate skin and also results in an optical shielding, thus preventing ambient light from penetrating into the region that is optically inspected with the sensor head. Accordingly, the measurement accuracy and reliability are improved as well as the ease of use of the sensor.

In addition, the proposed design of the sensor can allow, in particular, that a lower contact surface (defining said contact plane) of the sensor head and a lower surface of said cable can be arranged coplanar; i.e., the transition of the cable into the sensor head can be arranged completely flat / without a step, at least on the lower side that is facing the skin. Such a design offers significant advantages for the end user because pressure marks can be avoided on the skin, when the sensor head is brought into skin contact during a measurement. Moreover, the sensor can be brought much closer into contact, as compared to state-of-the-art designs.

For the same reasons, it is of advantage if the cable has a non-circular (e.g., approximately rectangular) cross-section, preferably with an aspect ratio (AR) of AR = width/thickness > 2:1, preferably with AR > 5:1.

We note at this point already, that the signals mentioned above, which are routed by said signal carrier(s), may be electrical currents/voltages and/or optical signals, depending on the sensor design / implementation; the invention is applicable in all such cases.

According to the invention, there exist further advantageous embodiments solving the afore mentioned problems, which are described in the sub-claims and in the following, and which may be also used in combination, to solve the afore-mentioned problem most effectively.

The optical sensor can be applied most conveniently on the small, strongly curved heads of neonates if the sensor head is designed such that the sensor head can be bent (repeatedly and without destruction) in a section corresponding to the middle portion of the at least one signal carrier. Due to the flexible nature and bending of the sensor head, the course of the smooth curve of the at least one signal carrier (which itself is preferably flexible) may thus be reversibly changed during use of the optical sensor (i.e., the smooth curve/the at least one signal carrier may thus be flexed and/or bent itself during use of the optical sensor). Such a design can be obtained most simply, if the sensor head is formed from a flexible material, for example silicone and also the at least one signal carrier is formed from a flexible material (e.g., the at least one signal carrier may be at least one flexible light guide or it may be formed by a flexible printed circuit board, as will now be detailed).

For example, one embodiment suggests that the at least one signal carrier is at least one electrical conductor. This at least one electrical conductor may be preferably formed by a flexible printed circuit board. Most preferably, the mentioned carrier substrate can be arranged on or formed by the flexible printed circuit board (i.e., the carrier substrate and the flex-PCB may thus be designed as one piece / formed as one single member, in particular; for example, the flex-PCB may be stiffened with additional FR4-layers at the distal end to form the carrier substrate in this area). In such a case, the at least one light source and/or said at least one detector may be optoelectronic components, which are mounted on the carrier substrate. Such a design is highly beneficial for achieving a high flexibility of the sensor head in the section corresponding to the middle portion of the at least one signal carrier/the flex-PCB.

Preferably, the lower contact surface (defining said contact plane) of the sensor head and a lower surface of said cable may be arranged coplanar. In such a design, these surfaces may still be bent into curved shapes, but they do not form a step. In other words, the lower contact surface and the lower surface of said cable may show a continuous transition. Preferably, at least in the area of the transition, the two surfaces may extend in a single (common) plane.

Alternatively, the at least one signal carrier may be formed by at least one light guide. Preferably, each of these light guides (if multiple guides are used) may be coated respectively by a separate cladding. In such a case, said at least one light source and/or said at least one detector can be formed by a respective distal optical element, for example. Such a distal optical element may be, for example, an end facet of a light guide or for example an optical mirror or an optical lens (which may receive light from a light guide and send the light out into tissue). Again, by using (thin) flexible light guides, the course of the middle portion can be easily adapted/flexed during use of the optical sensor.

In the case of using light guides as signal carriers, at least one optoelectronic source of light (e.g., an LED) and at least one optoelectronic detector unit (e.g., a photodiode) may be arranged at a distal end of said cable, either as part of the sensor or as part of a separate electronic module. The light from the optoelectronic source of light may be guided by the at least one light guide to the light source located in the head of the sensor. The latter thus not necessarily produces the light but emits it from the sensor head. For example, the at least one light source located in the sensor head may be functionally coupled to lenses or other optical means for forming a beam of light to be sent out from the head of the sensor. Another one of the at least one light guide may be used to guide light which is received by the detector (in the simplest case an end facet of the respective light guide) located in the sensor head to the optoelectronic detector unit located at the proximal end of the cable.

Most preferably, the least one signal carrier, in particular said printed circuit board, may be supported by or suspended by at least one flexible supporting structure at the location of the smooth curve mentioned before. Most preferable are designs in which the supporting structure is bendable/flexible around a transverse axis (x-axis) of the sensor, because this enables a close and large area skin contact of the sensor.

According to one particular embodiment, the flexible supporting structure may be formed by at least one pillar, which can be provided by the sensor head. It is also possible that the at least one pillar extends from the location of the smooth curve. In this case, the at least one pillar can be embedded in a wall portion of the sensor, for example. When using two or more pillars, the pillars may be separated by gaps from each other. Such a design is beneficial for achieving a high degree of flexibility of the support structure.

The at least one pillar may have a round or rectangular shape, for example; the at least one pillar may also extend longer in a direction perpendicular to the main routing direction (x-direction) of the at least one signal carrier than in said main routing direction (y-direction), which is beneficial for achieving high flexibility for bending around the transverse x-axis

According to one embodiment, the at least one pillar is formed by a lower member of the sensor head, which defines said contact plane.

Alternatively, the at least one pillar may be formed by a molded member. In other words, at least one pillar may be formed separately from said lower member forming part of the sensor head.

One design suggests that a first distal pillar which is provided by the sensor head and which supports said at least one signal carrier (i.e., in particular said flexible printed circuit board) in the middle portion shows a distance D2 from a proximal end of the carrier substrate of D2 ≥ 0.20 mm. Preferably, D2 may additionally such that D2 ≤ 0.60 mm. Such a design results in a compact sensor head and at the same time, efficient underfilling / embedding of the signal carrier / the flex-PCB can be guaranteed, in particular when using vacuum molding.

To improve the optical functionality of the sensor, it is of advantage if the elevation D1 of the carrier substrate from the contact plane is D1 ≥ 1.0 mm. This also results in a bending that can be repeatedly defined accurately.

The mentioned at least one pillar can be a molded member. In this case, it is preferred, if the at least one pillar is molded from a flexible material such as silicone or is formed by an injection molded member. For example, the at least one pillar may be molded into a recess of a wall portion of the sensor.

The sensor head, preferably a lower member of the sensor head, may carry at least two pillars. These at least two pillars may extend to the at least one signal carrier. In particular, the at least two pillars may support the at least one signal carrier (i.e., in particular said flexible printed circuit board) in the middle portion. In such a case, each of said at least two pillars may provide a respective supporting surface which follows the respective smooth curve of the respective middle portion of the at least one signal carrier. As a result, the supporting surfaces may define said smooth, in particular S-shaped, curve. In particular, the respective supporting surface may be curved and hence non-planar.

The lower member of the sensor head mentioned before can be a molded member, for example an injection molded member. Moreover, the lower member may forms said at least two pillars. Hence, the at least two pillars and the lower member may be formed as one piece.

Alternatively, said at least one pillar can be a molded member but can be fabricated separately from said lower member. For example, during molding of the sensor head, the middle portion of the flexible printed circuit board may be temporarily supported by removable metal pillars. After removing these pillars or pins (which may be part of a mold using during the molding step), the resulting voids (at the locations of the pins) below the middle portion of the flex-PCB may be filled up with a casting compound (which may be the same or a different material than that used for molding the sensor head/said lower member) to form molded pillars supporting the middle portion of the flex-PCB in the final sensor device. It is clear, that in this case a boundary may be optically perceivable between the molded member and the molded pillars. These molded pillars may thus form the flexible supporting structure, which supports the smooth curve of the at least one signal carrier, as described above with respect to the optical sensor.

The at least two pillars, in particular said respective supporting surfaces, can also each extend longer, preferably at least 5 times longer, along a transverse axis of the sensor then along a longitudinal axis of the sensor. The longitudinal axis may coincide with a main routing direction of said signal carrier. Such a design provides high robustness to the sensor and at the same time allows sufficient bending in the area of the smooth curve along the transverse axis.

For example, the at least two pillars may be separated from each other along a longitudinal axis of the sensor by interspaces, which are running along a transverse axis of the sensor. Preferably, the transverse axis may extend in a plane that is parallel to a plane defined by the carrier substrate. These interspaces may be air-filled; preferably, however, the interspaces may be filled, at least partly, with a moldable material, in particular a casting compound to increase the robustness of support for the signal carrier. The at least two pillars can also be arranged in a 2-dimensional array with such interspaces running along the transverse axis and along the longitudinal axis of the sensor.

With respect to the smooth curve mentioned before, it is noted that this curve may feature an inflection point indicating a transition of a course of the at least one signal carrier (i.e., in particular of said flexible printed circuit board), from a left-handed bend into a right-handed-bend (or vice versa). In this case, it is preferred for accurate definition of the curve, if the at least one signal carrier, in particular said flexible printed circuit board, is supported by a pillar at the location of the inflection point.

In addition, or alternatively, it is also preferably, if the at least one signal carrier (in particular said flexible printed circuit board), is supported by respective pillars, which are located upstream and downstream of the location of the inflection point, respectively. In other words, from the middle portion onwards the at least one signal carrier, in particular said flexible printed circuit board, may thus rise monotonously above the contact plane to the carrier substrate (w.r.t. to said contact plane).

The sensor head can feature a lower member (which may be, in particular, said lower member mentioned above), which forms a contact surface to be brought into skin contact during a measurement. Preferably, the sensor head design may be such that this contact surface is bendable. In this case, the sensor head can be brought into large-scale skin contact on variously sized skulls (with different curvatures). A center area of said contact surface may coincide with said contact plane mentioned before. The contact surface may also reach into a section of the sensor head corresponding to the middle portion of the at least one signal carrier, such that this section of the sensor head can also be brought into skin contact during a measurement.

In a relaxed state, the contact surface can thus be planar, or it may show a (pre-defined but possibly changeable) curvature around a transverse x-axis of the sensor. In the latter case, it is preferred if the contact surface shows a further curvature along a longitudinal y-axis of the sensor. As will become evident from the Figures, the longitudinal y-axis of the sensor may be defined by the course of the cable, whereas the transverse x-axis may run perpendicular to the longitudinal axis and perpendicular to the z-axis along which the sensor emits and receives light during a measurement.

The carrier substrate may be stiffer than the flexible printed circuit board. For example, it can be attached to two sides of the printed circuit board as a stiffener structure.

The cable may be designed as a flat band. In this case, the flexible printed circuit board may run most preferably in a middle plane of the band.

The cable of the sensor and/or said lower member of the sensor head may be bendable in such a way that a proximal portion of the at least one signal carrier (i.e., in particular of said flexible printed circuit board), can be arranged such that the proximal portion runs parallel to said contact plane; alternatively, the bending may be such that in a relaxed state, the proximal portion may run obliquely and/or curved to said contact plane.

Preferably, the middle portion of the signal carrier / of said flex-PCB can run at an angle α of at least 20° to said contact plane in one portion. Moreover, at least initially on the proximal side, the middle portion may extend parallel to said contact plane in another portion.

The carrier substrate can be supported by supporting members provided by the sensor head, preferably provided by said lower member. In addition, the carrier substrate can be embedded in a moldable material, in particular in said lower member of the sensor head (in case said member is fabricated as a molded member).

The sensor head, in particular said lower member, can be bendable. This may be achieved by form the head / said lower member from a flexible material, preferably from a silicone.

The at least one light source and/or said at least one detector may be encased by at least one metal housing. In this case, the at least one metal housing may be fixed to the carrier substrate, preferably by a solder connection or by gluing. Moreover, the at least one metal housing may be supported by said lower member of the sensor head. Such a design can result in efficient electromagnetic shielding.

For solving the afore-mentioned problem, the invention also suggests a method for fabricating a sensor, in particular according to one of the claims directed towards an sensor or as described herein. In other words, the sensor to be fabricated may comprise: a sensor head bearing at least one light source and at least one detector, which are arranged on a carrier substrate (in particular, respectively). This sensor head may define a contact plane or contact surface to be brought into skin contact during a measurement. The sensor may also comprise a cable featuring at least one signal carrier configured to route signals to and/or from the sensor head.

According to the invention, the method comprises the following steps for solving the problem: a carrier substrate is arranged on a level which is elevated above the contact plane by a distance D1; a middle portion of the at least one signal carrier, which is located between the carrier substrate and a proximal portion of the at least one signal carrier, is bent into an smooth curve; this may be achieved by using temporarily support structures for example, or by using the mentioned at least one pillar directly. Finally, the smooth curve is fixed by embedding the middle portion into a mold material.

This method can be further elaborated, as will now be explained: For example, prior to molding, a pre-fabricated lower member of the sensor may be arranged below the middle portion to define the smooth curve and/or to achieve the desired bending of the middle section.

Alternatively, it is also possible that, during the molding, temporary support structures (which do not form parts of the fabricated sensor later) are used to define the smooth curve temporarily. In this case, the temporary support structures may be removed after the molding to form voids and the voids can then at least partly be filled up with a mold material in a second molding step. This way, a strong support structure for the signal carriers can also be fabricated.

In both cases (using permanent or temporary support structures during molding), the smooth curve may also be maintained in position and shape by clamping from two sides. I.e., two (preferably flexible and permanent or temporary) support structures may be used on both sides of the smooth curve to clamp the middle portion of the at least one signal carrier (in particular of said flexible printed circuit board). If temporary support structures are used, the clamping may be removed after molding, and the remaining voids may be filled up with a molding compound afterwards (at least partly).

Finally, during the molding, a supporting structure (in particular as described in detail before w.r.t. the design of the sensor, i.e., for example a supporting structure formed by at least one pillar) forming part of the fabricated sensor can be used to define the smooth curve.

Preferred examples of the present invention shall now be described in more detail, although the present invention is not limited to these examples: for those skilled in the art, it is obvious that further examples of the present invention may be obtained by combining features of one or more of the patent claims with each other and/or with one or more features of an example as described or illustrated herein.

With reference to the accompanying Figures B1 to B8, where features with corresponding technical function are referenced with same numerals even when these features differ in shape or design:
- Fig. 1: shows a perspective side view on inner components of an optical sensor according to the invention,
- Fig. 2: shows the same optical sensor in a cross-sectional view along a longitudinal y-axis,
- Fig. 3: shows a detail of the cross-sectional side view of Figure 2,
- Fig. 4: shows a lower member of the head of the sensor of Figure 1,
- Fig. 5: shows the lower member of Figure 4 together with a flexible printed circuit board and attached carrier substrate,
- Fig. 6: shows a detail of a cross-sectional side view of another optical sensor according to the invention, in which the middle portion also follows a smooth curve,
- Fig. 7: is a schematic illustration to show the contact plane defined by the head of the sensor and the curvature of the sensor head`s contact surface, and
- Fig. 8: shows the optical sensor of Figures 1-3 in a bended state, in which the cable 3 has been bended downwards.

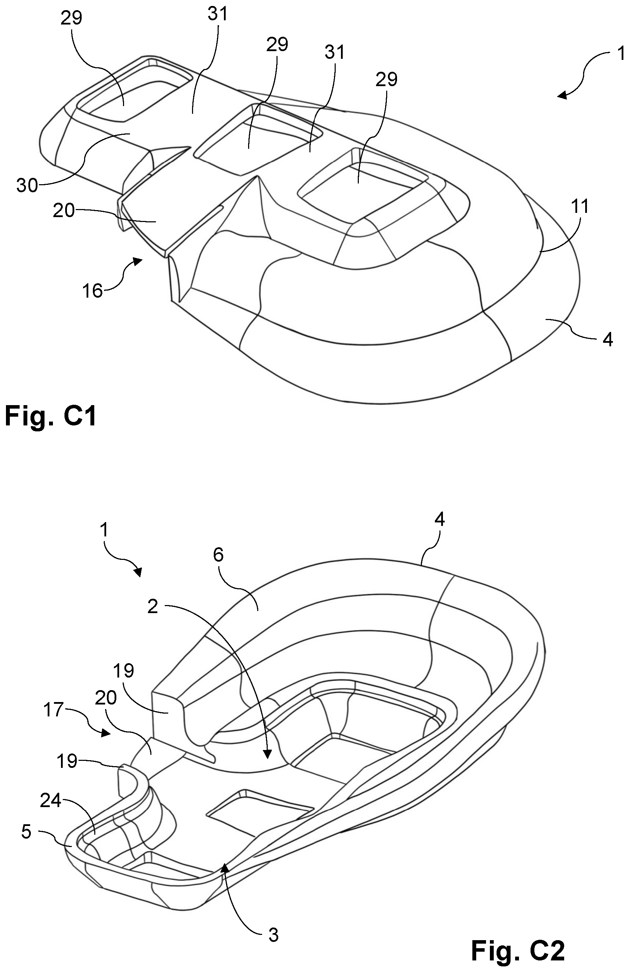

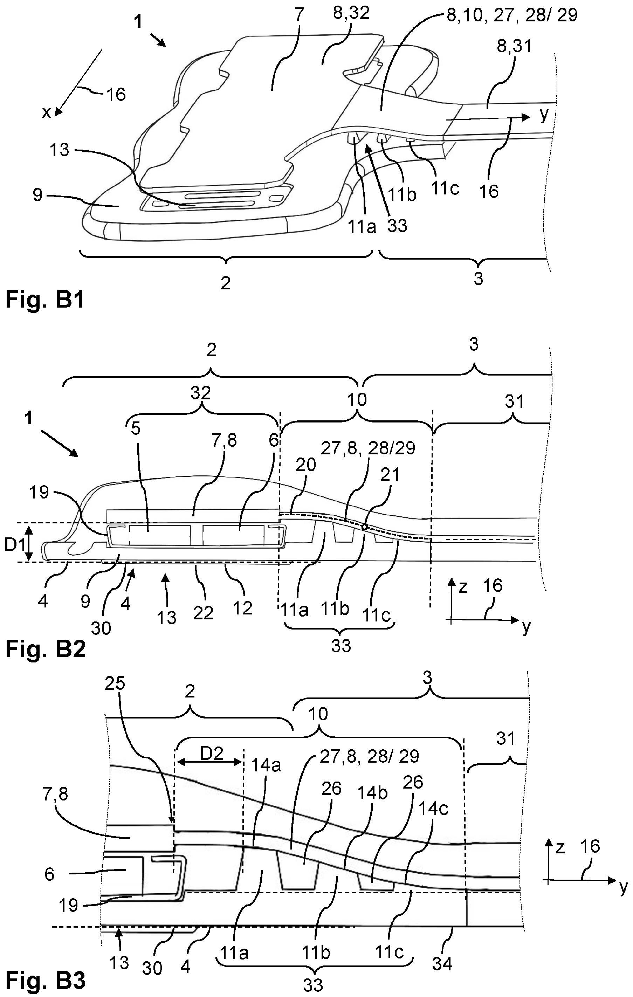

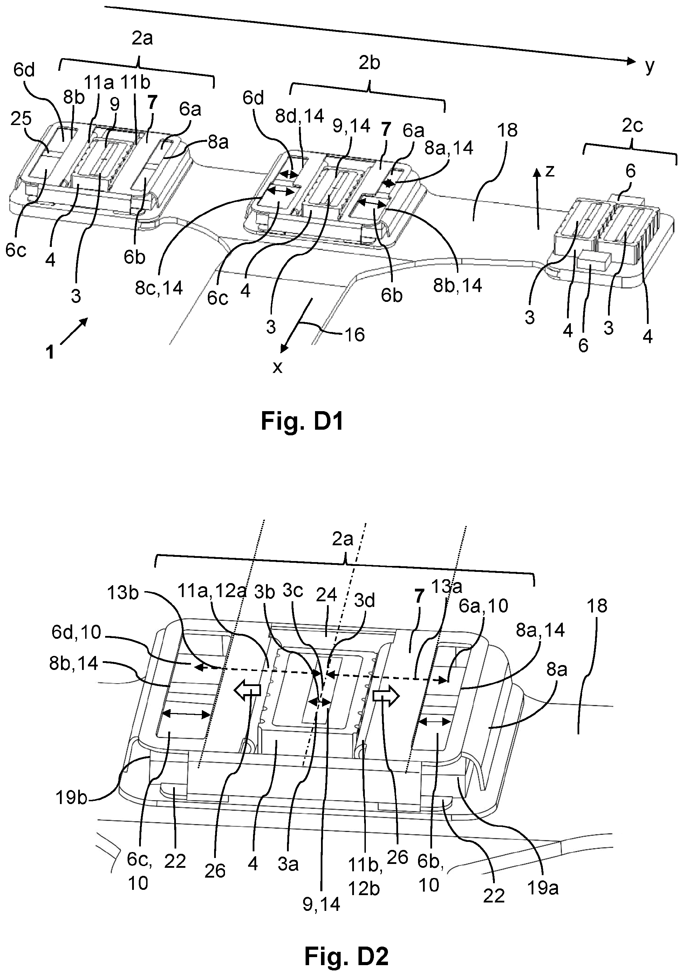

Figure 1 shows a perspective side view on inner components of an optical sensor 1 according to the invention, which is also depicted in Figure 2. The optical sensor 1 is designed as a near infrared spectroscopy (NIRS) sensor and can optically measure oxygenation levels in subcutaneous tissue layers, in particular in cerebral tissue, for vital parameter monitoring. For that purpose, the sensor 1 is brought into skin contact with its lower side, which forms a soft contact surface 12 (cf. Figure 2), on the skull of a patient. IR-light emitted by light sources 5 of the sensor 1 is then irradiated into the tissue (along the z-direction in Figure 2) and the part of the IR-light which is back-scattered by the tissue is collected by the sensor 1 using several detectors 6 located at different source-detector-separations (SDS) from the respective light source 5.

As illustrated in Figure 2, the optical sensor 1 features a sensor head 2 which bears several LEDs as light sources 5 and several photodiodes (PDs) as detectors 6. These optoelectronic components 5,6 are arranged on and electrically connected to a carrier substrate 7 in the form of stiff printed circuit board (PCB) formed from FR4-material and copper conducting lines. The carrier substrate 7 is electrically connected to a second flexible PCB 8, which features several copper conductive tracks as electrical conductors 28 that are running along the longitudinal direction 16 (y-axis) of the sensor 1. Part of the flex-PCB 8 is embedded in a flat cable 3 formed from silicone, with the flex-PCB 8 running in a middle plane of the band formed by the flat cable 3. The conductive tracks of the PCB 8 form signal carriers 27, which serve to route electrical signals to the sensor head 2 to drive and control the operation of the light sources 5 and detectors 6 and to route electrical signals from the sensor head 2 to read-out measurement signals delivered by the detectors 6.

We note at this point that the concept presented herein can also be implemented by replacing one or more of the conductive tracks by light guides 29 as signal carriers 27 (as indicated by the reference numeral 29 in Figures 1 and 2). In this case, an electronic source of light may be used as an external component to deliver optical signals through the light guides to the sensor head 2. In this case, the LEDs may be replaced by light sources which can be formed, for example, simply by end facets of the light guides, to send out light from the sensor 1. In the same manner, other end facets of light guides may be used as detectors to receive the back-scattered light and send these optical signals via the light guide (along the cable) to an electronic detector, which may also be an external component.

As is visible in the side-view of Figure 2, the carrier substrate 7 is elevated above the contact plane 4 (defined by the lower surface of the sensor head 2) by a distance D1 of more than 1 mm. This is necessary to allow beam shaping of the light send out by the LEDs 5. A metal housing 19 is mounted on the carrier substrate 7 to shield the LEDs 5 and PDs 6 from electromagnetic interference. As visible, the carrier substrate 7 rests via the metal housing 19 on a lower member 9 of the sensor head 2, which has been formed by injection molding.

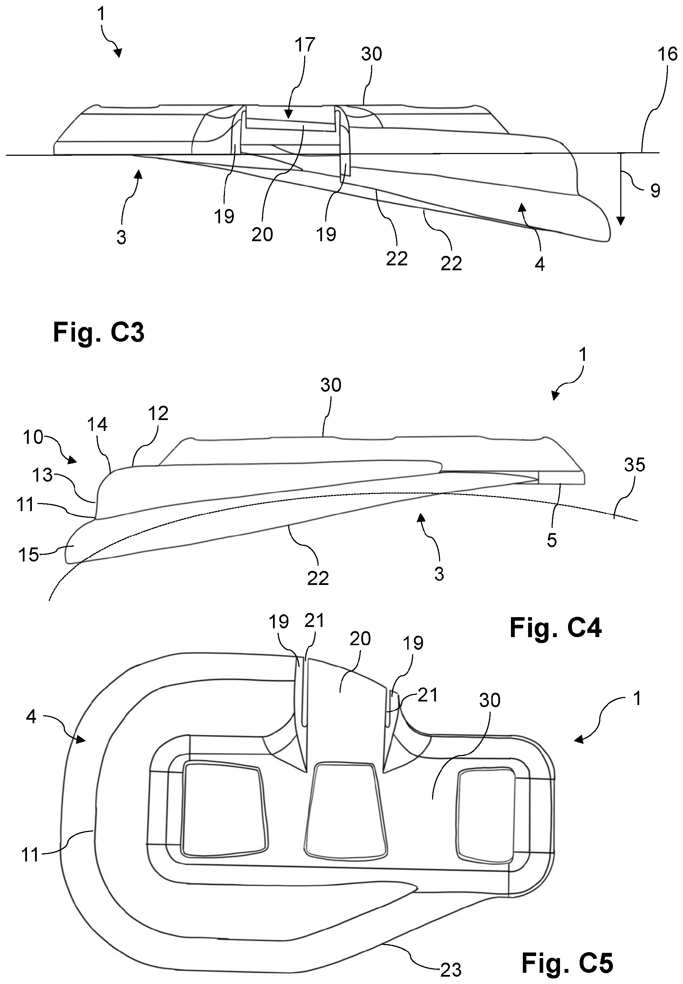

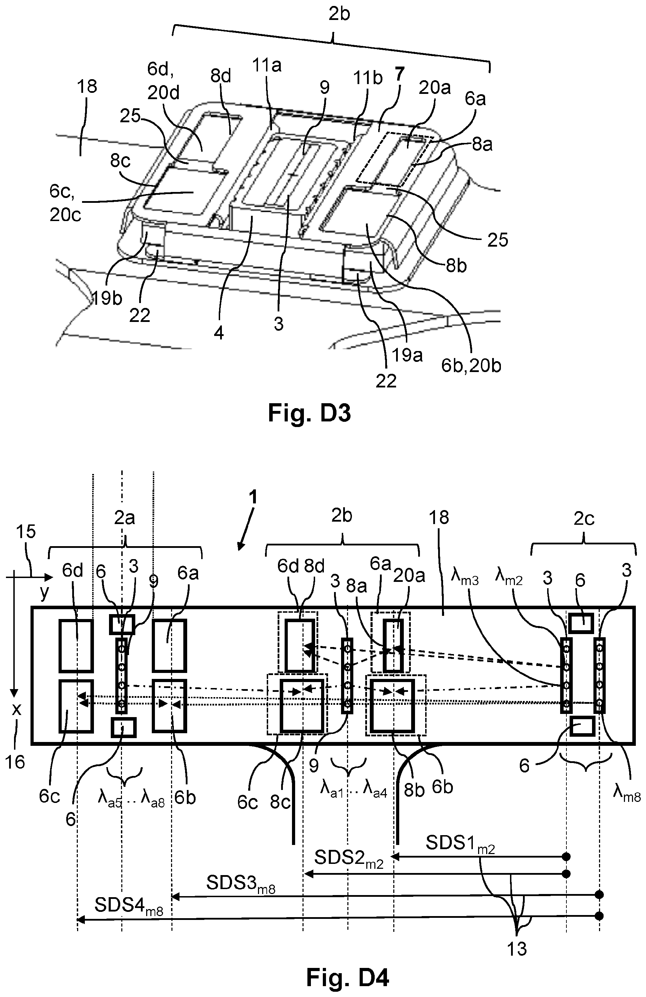

In Figures 1 and 2 it is visible that a middle portion 10 of the flex-PCB 8 (which serves as the signal carrier 27) follows a smooth curve 20. As visible from the more detailed view of Figure 3, this middle portion 10 is located between the carrier substrate 7 (on the left/at the distal end of the flex-PCB 8) and a proximal portion 9 of the flex-PCB 8, which is embedded in the flat cable 3.

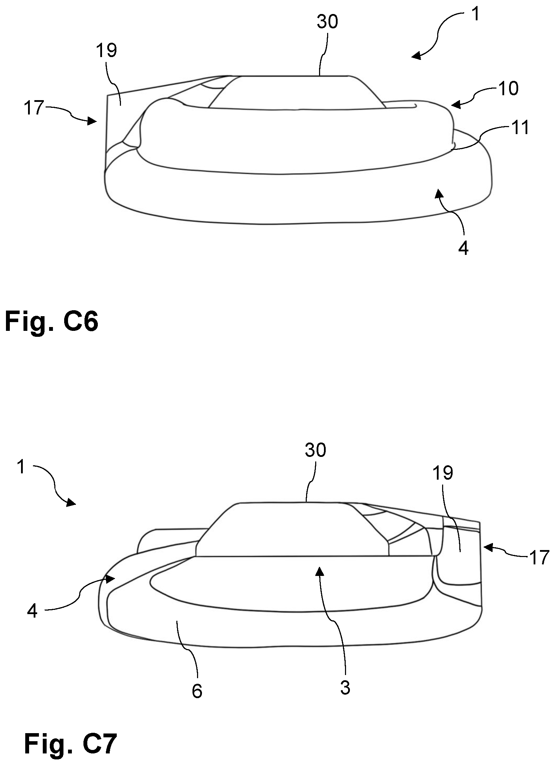

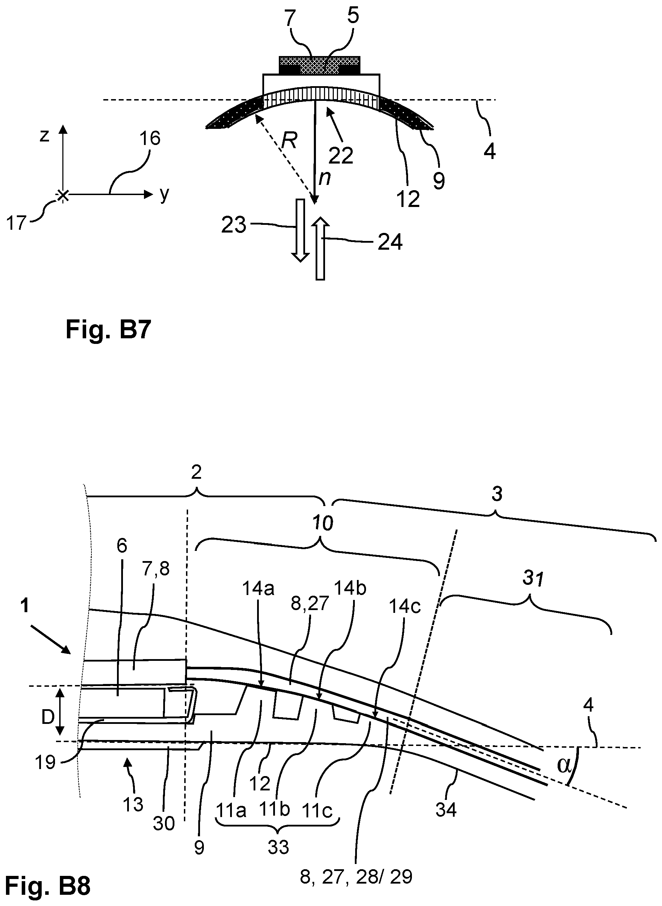

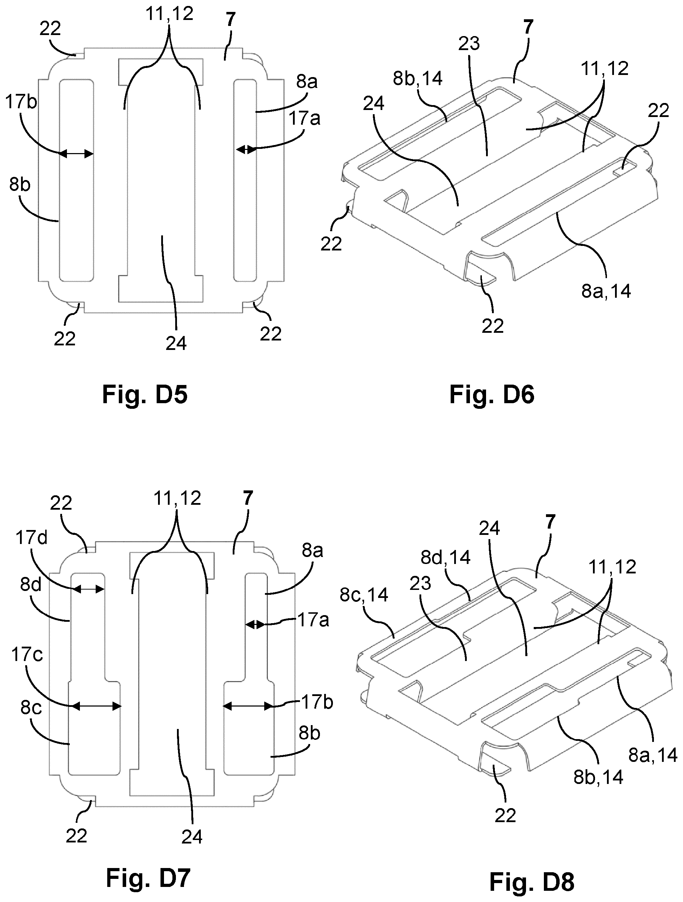

As shown in Figure 1-3, a lower member 9 of the sensor head 2 forms three pillars 11a, 11b and 11c, which form a supporting structure 33 for supporting the flex-PCB 8 at the location of the smooth curve 20 (mainly during manufacturing and prior to molding). As has been mentioned before, the injection-molded and flexible lower member 9 also defines the contact plane 4 that is to be brought into skin contact during a measurement, i.e., it forms a skin interface. More precisely, the lower member 9 forms a bendable contact surface 12 to be brought into skin contact during a measurement and the center area 22 of said surface 12 coincides with the (imaginary) contact plane 4 mentioned before - see for example Figures 2 and 7.

Also note that the middle pillar 11b supports the flex-PCB 8 at the location of an inflection point 21 of the smooth S-shaped curve 20, whereas the distal pillar 11a and the proximal pillar 11c are located upstream and downstream (seen from the perspective of signal routing from the head 2 to the proximal side of the cable 3) from the inflection point 21, respectively. As a result, the signal carrier 27/the flex-PCB 8 rises monotonously above the contact plane 4 to the carrier substrate 7 when following the middle portion 10 onwards to the carrier substrate 7. At the same time, the proximal portion of the flex-PCB 8 just to the right of the middle portion 10 can be bent such that it runs parallel to the contact plane 4, as is illustrated in Figures 2 and 3.

An alternative for achieving a suitable support structure can be to only temporarily support the middle portion 10 of the signal carrier 27 during fabrication of the sensor 1 using, for example, retractable metal pins. This also allows definition of the intended smooth curve 20 of the middle portion, which may then be fixed by embedding the middle portion 10 in a molding compound. Afterwards, the metal pins may be removed, and the resulting voids may then be filled up (at least partly) during a second molding step. In this case, the mold material filled into the voids will form the desired final support structure for the smooth curve 20. In this case, the pillars 11 can thus be molded from a flexible material such as silicone and they can be fabricated separately from the lower member 9.

As Figure 3 shows, the most distal pillar 11a shows a distance D2 from a proximal end 25 of the carrier substrate 7. This distance is chosen to be larger than 0.2 mm and smaller than 0.6 mm. Accordingly, a sufficiently large gap between the distal pillar 11a and the carrier substrate 7 is formed that can be filled with a molding compound. At the same time, the gap is sufficiently small such that the sensor head 2 remains compact and the S-shape curve 20 is accurately controlled.

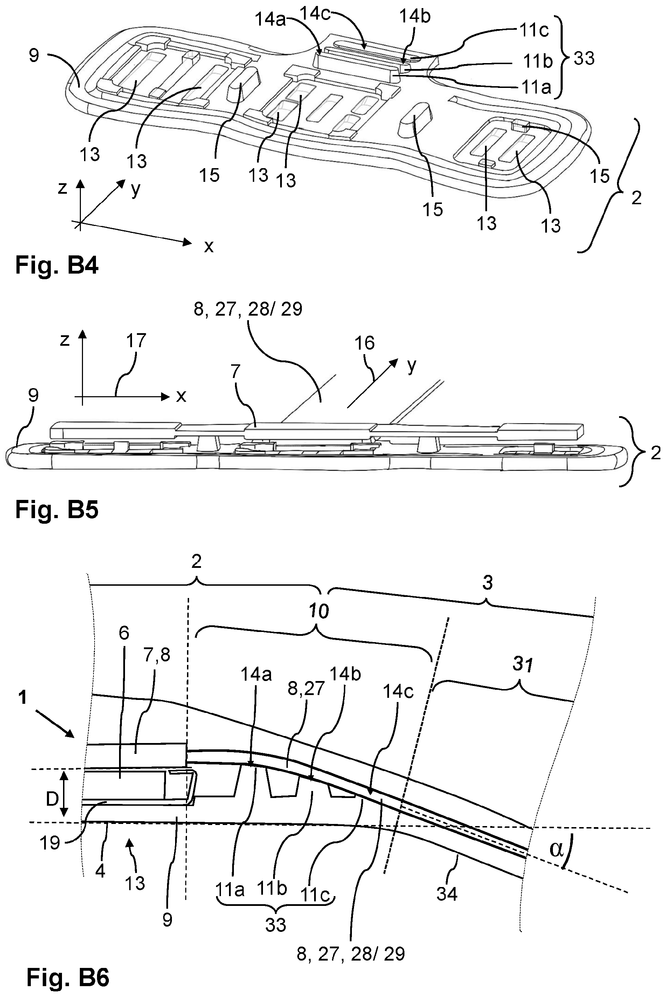

As is revealed by the detailed view of the lower member 9 in Figure 4, the three pillars 11a/11b/11c each extend longer (in fact more than 5x longer) in the transversal x-direction as in the longitudinal y-direction of the sensor. Each pillar 11 forms thus an approximately rectangular supporting surface 14a/14b/14c. These surfaces 14 are non-planar and curved and thereby define the smooth curve 20 of the middle portion 10 of the signal carrier 27 / the flex-PCB 8. It is also visible that the pillars 11 are separated from each other along the longitudinal y-axis 16 by interspaces 26 running along the transverse x-axis 17 (cf. Figure 4 with Figure 1).

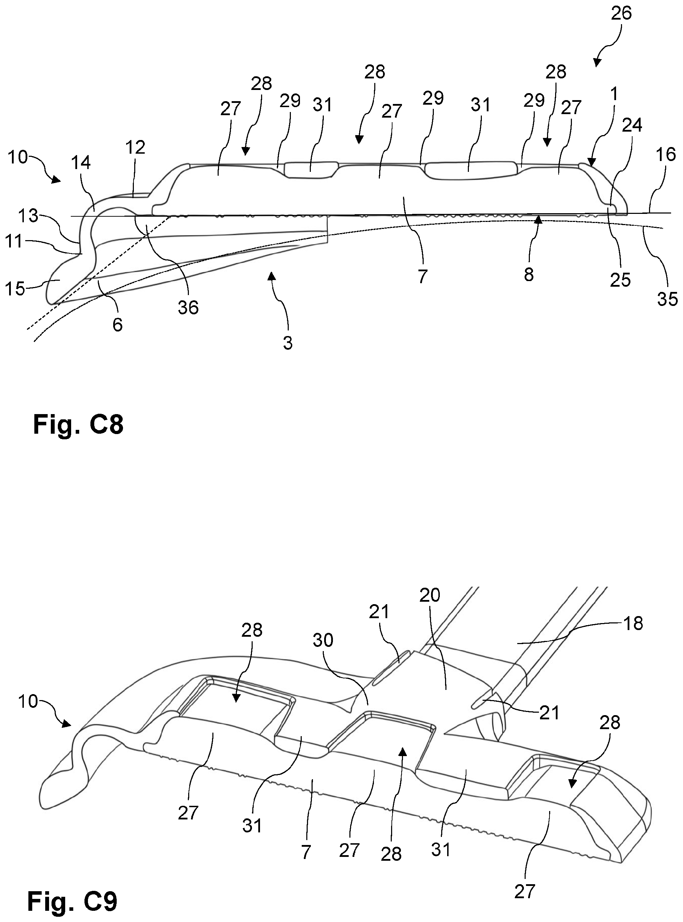

As can be seen in Figure 2 and 3, the lower contact surface 12 of the sensor head 2 and a lower surface 34 of the cable 3 are arranged coplanar. Nevertheless, these surfaces 12, 34 may also be bended or designed with a curved shape as shown in figures 6 and 8, but they do not form a step. In other words, the lower contact surface 12 and the lower surface of the cable 3 show a continuous transition, i.e., they merge smoothly into each other without forming a gap or step. Preferably, both surfaces 12, 34 may be formed by a common member as one piece. Also, these two surfaces 12, 34 may extend in a single/common plane, at least in the area of the transition.

In Figure 4, we can also see respective windows 13 (formed by a transparent silicone), which are formed by the lower member 9, whose main body is non-transparent for the NIR-radiation sent out by the light sources 5. Also note the supporting members 15, which serve the purpose of defining the elevation D1 of the carrier substrate 7 above the contact plane 4, as illustrated in Figures 5 and 2.

As shown in figure 4, light sources may be located adjacent to windows 13 on the right side in the drawing, while the detectors may be located adjacent to the windows 13 on the left side and in the middle of the drawing. Of course, other placements of the light sources and or detectors can also be implemented while maintaining the design according to the invention.

Figure 6 shows an alternative design of an optical sensor 1 according to the invention, which also features a signal carrier 17 that is bent into a smooth curve 20 and supported by at least two pillars 11. The design differs, however, from that of Figures 1 to 5 in that - in a relaxed state (i.e., without external stress applied) the proximal portion 31 of the signal carrier 27/the flex-PCB 8 runs obliquely w.r.t. the contact plane 4. In other words, in this design, the middle portion may also be bended into a smooth curve but without external forces applied, the middle portion will run along a smooth curve as shown in Figure 6, which does not resemble an "S". Such a design can help to avoid the formation of pressure marks, when attaching the sensor 1 to the small heads of neonates. It is also possible to form the lower member 9 in such a way that the contact surface 12 can actually be non-planar and show a curvature R, for example along the transverse x-axis 17 as depicted in Figure 7. Also note that the lower member 9 may actually be embedded in an outer soft shell (e.g., a silicone layer) of the sensor head 2. In the same manner, the carrier substrate 7 may also be embedded in a moldable material.

Figure 8 shows the optical sensor 1 of Figures 1-3 in a bended state, in which the cable 3 has been bended downwards such that the curve, which the middle portion 10 follows, now deviates from the initial S-Shape. Due to the flexible nature of the supporting structure 33, which comprises the three pillars 11a, 11b, and 11c (note the deformation in particular of the first pillar 11a), the middle section 10 of the flex-PCB 27 has thus adjusted its smooth course, as can be seen be comparing Figure 8 with Figure 3. As is thus illustrated, during use of the optical sensor 1, the middle section 10 may be flexed and bended easily and as a result, the lower contact surface 12 of the sensor 1 can be brought into conformal skin contact even on highly curved skin surfaces.

In summary, for improving the conformability of a head 2 of an optical sensor 1 and also to improve shielding from ambient light, and for avoiding pressure marks on skin, a novel design of an optical sensor 1 is suggested which features a signal carrier 27, for example in the form of a flexible PCB 8 or in the form of a bundle of light guides 29, which is formed into a smooth continuous curve 20 by a supporting structure 33 that preferably features at least two pillars 11. The support structure, which may still be bendable and allow deformation of the smooth curve 20, helps in guiding the signal carrier 27 in a way that avoids the formation of pressure spots; it also helps to bring a lower contact surface 12 of the sensor`s head 2 into close vicinity to the skin during a measurement (cf. Figure 2). Through these measures, more reliable optical measurements can be obtained and the comfort for the patient is also improved.

In another **aspect C)**, the invention concerns a light shielding cover for an optical sensor for measuring optical parameters in a scattering medium, wherein an active surface of the optical sensor is placed on a surface of the medium during measuring.

The invention relates to the field of spectrophotometry and oximetry, which aims at measuring optical properties and deriving parameters such as oxygen saturation in living tissue. Spectrophotometry allows determination of optical properties such as absorption and scattering coefficients by illuminating a specimen and determining the attenuation in light intensity over distance. Oximeters are apparatuses that make use of spectrophotometry for determining the (arterial, venous or mixed) oxygenation of blood in tissue.

In particular, the invention relates especially to the measurement of cerebral hemoglobin saturation in heads, especially of neonates or small children. The heads of neonates or small children are very small and therefore have a small radius and strong curvature. It is thus necessary to fix the active surface of the sensor to the head using a bandage or other means. But neonates also have very thin and sensitive skin. When fixing the sensor to the head, excessive forces and pressure on the skin should be avoided.

It is an object of the present invention to improve usage properties of an optical sensor.

In accordance with a first embodiment of the present invention, the object is solved by a light shielding cover according to claim 1.

According to the invention the light shielding cover comprises a chamber for inserting an optical sensor, wherein the chamber comprises an opening through which the active surface of an inserted optical sensor is accessible, and a sealing lip surrounding the chamber, wherein the sealing lip protrudes from the chamber in a direction normal to and away from the active surface of an inserted sensor (towards the patient) and wherein the circumference of the sealing lip at the distal portion forming a contact area is larger than the circumference of the chamber.

The sealing lip may be formed adjacent to a rim of the chamber and thus protrude from that rim.

Such an optical sensor comprises in general one or more light emitting sources and one or more light receivers, placed distanced away from the light emitting source. To perform a measurement, a sensor is inserted into the chamber of the light shielding cover according to the invention. The sensor with the cover is then placed on the surface of the medium to be measured. The medium could be for example cerebral tissue of a neonate, in which case the sensor with cover is placed on the head of the neonate.

When placed on the surface, for example the head of a neonate, the active surface of the sensor is brought into contact with the surface of the medium through the opening in the chamber. The sealing lip, which protrudes away from the active surface, that means in the direction towards the surface of the medium, tightly seals the chamber and shields the sensor from ambient light. Thus, the sensitivity and accuracy of the measurement can be increased since the signal to noise ratio is increased.

Even on surfaces with small radii, such as for example a head of a neonate, where a sensor would be strongly curved and may not fit snugly on the head, the sealing lip will tightly shield the sensor from ambient light.

Since the circumference of the sealing lip is larger than the circumference of the chamber, and an inserted sensor, it also reduces the amount of light that reaches the sensor(s) by entering the skin next to the sensor and percolating the tissue or skin to the sensor.

The light shielding cover has the overall effect, that less force is needed to fit it tightly to the surface. The usage properties, when using the invention on neonates is thus improved.

In accordance with another embodiment of the present invention, the object is solved by a light shielding cover according to claim 2.

In the embodiment the cover comprises a chamber for inserting an optical sensor, wherein the chamber comprises an opening through which the active surface of an inserted optical sensor is accessible, wherein the chamber has one or more relief portions to accommodate protrusions or stiffer portions of an optical sensor, wherein a relief portion comprises a recess in the inside and/or the outside of the chamber wall associated with the location(s) of the protrusion(s) or stiffer portion(s) of an inserted sensor.

As described earlier, a sensor comprises at least a light source and a receiver. These parts are usually stiffer than for example a surrounding portion of the casing and they directly or indirectly (via for example a filter or window) contact a surface of a medium. That means, when applied to for example the head of a neonate, these stiffer portions can cause pressure points on the skin of the head which may lead to damage of the skin.

The relief portions have the effect, that pressure applied to the cover, for example by a fixating bandage, is not directed to these stiffer portions of the sensor. This greatly reduces or eliminates indentations of the stiffer portions into the skin. Neonates normally are monitored during longer periods so that the elimination of such indentations is a vast improvement of the usage properties of a sensor.

In an aspect the chamber has a counterpart form of an optical sensor to be housed. This has the advantage that the cover sits tightly on the sensor and pressure applied on the cover is well distributed and passed on to the sensor. Therefore, a tight seat on a surface of the sensor and the cover is provided. Moreover, if relief portions are present, the pressure applied on the cover is distributed to non-protruding portions and or less stiff portion(s) of the sensor.

Alternatively or additionally, the recess of one or more relief portions comprise a through opening in the chamber wall. Preferably any protrusion or stiffer part of the sensor does not project from the opening to the outside of the chamber. This has the effect that any fixation means, like a bandage, does not apply pressure on the stiffer part(s). The risk of excessive pressure or the forming of indentation on the surface of the skin is reduced.

In one aspect, the sealing lip comprises a bending area. The bending area allows the sealing lip to be compresses or at least deflect or move more easily relative to the chamber. Preferably the deflection is facilitated in a direction normal to a surface where the sealing lip is placed on. However, other deflecting directions could be possible. The bending area thus enables the sealing lip for example to adapt to different shapes, sizes and curvatures of heads and provides tight shielding from ambient light. It is possible that the sealing lip comprises more than one bending area.

Preferably the sealing lip extends circumferentially around at least a portion of chamber, preferably around at least 25% of the circumference of the chamber.

Preferably the bending area extends circumferentially around at least a portion of chamber, preferably around at least 25% of the circumference of the chamber and or the bending area extends circumferentially around only a portion of the circumference of the chamber, preferably around less than 75% of the circumference of the chamber.

In an example, the sealing lip comprises a circumferential step. The step can constitute or be part of the bending area or it can be distal thereof.

In another example, the sealing lip has an S-shaped cross section, wherein at least one bend of the S-shape constitutes the bending area. Preferably the other bend of the S-shape is part of the sealing lip.

As another example, the sealing lip is formed in the form of a gaiter or bellow, wherein the S-shaped cross section could provide a gaiter function.

The above-mentioned examples merely describe some possible embodiments of the sealing lip without the invention being limited to the examples. Also, these examples are not mutually exclusive but could be combined in any way.

Typically, a sensor has a rectangular shape with a long side and a short side. Axial in the context of the invention means parallel to the long side of the sensor.

In an aspect, the contact area of the cover has a concave cross section, at least in an axial direction of the cover form. The concave form enables the cover to fit on small objects or surfaces with strong curvature and still provides shielding from light.

In an aspect, the toe of the sealing lip is inclined with respect to the plane of the active surface of a sensor, especially wherein the inclination angle is between 20 and 60 degrees, preferably tapering in radial direction of the cover towards the sensor. The inclined surface provides a larger contact area on a surface and thus provides better shielding. It also provides softer skin contact. The inclination angle may be adapted to different curvatures of the body part. It may be advantageous to provide different covers with varying inclination angles for different curvatures. Therefore, a cover may be selected according to an actual use case.

In an aspect, the sealing lip comprises a chamfer in axial direction or a circumferential direction, especially wherein a height of the sealing lip is essentially zero at the base of the chamfer. A circumferential direction is a direction parallel to a side of the chamber. That means the sealing lip varies in its height along such a direction. The hight is measured in the direction normal to the rim of the chamber.

It is especially beneficial, if the chamfer is oriented in a way that the sealing lip has its maximum height in a region where a receiver of an inserted sensor is seated. Accordingly in a region where a light source of an inserted sensor is seated, the height of the sealing lip could be zero since no light shielding is required there for proper measurements.

Due to the chamfer in the sealing lip, the cover is less stiff in an axial direction and facilitates the sealing of small heads or strong curvatures. Moreover, if the sealing lip does not run completely around the chamber, the pressure required to form or fit the chamber to the patient is further reduced.

The chamfer also saves material and the cover may be cheaper to manufacture.

In an aspect, the sealing lip is asymmetrically arranged around the chamber. That means the sealing lip does not fully encircle the circumference of the chamber. In other words, there is at least a region in the circumference of the chamber, where the height of the sealing lip is zero or nearly zero.

This may provide better sealing on surfaces with strong curvature. The asymmetrical sealing lip can be formed by a chamfer on one or both long sides of the chamber in an axial direction. This is especially beneficial when the sensor is also asymmetrical, that means when the light sources are placed on one side of the active surface only.

In one aspect, the chamber comprises a circumferential recessed rim where a counterpart rim of an optical sensor bears on. This has the effect that the cover has a surrounding contact area to the sensor which may improve or enable a uniform pressure distribution.

In an aspect the cover comprises means for fixing a sensor in the chamber. By this the sensor is held in the cover even when not fixed to a surface. Such fixing means may improve the handling and usability of a sensor.

In an aspect, the cover comprises as fixing means an undercut where a rim of the sensor could be inserted or that embraces a part of the sensor.

In another aspect, the cover comprises as fixing means a strap that spans between two opposing sides or rims of the chamber. Preferably the sensor comprises a trench or recess where this strap is seated when the sensor is inserted in the chamber, so that the strap does not project from the plane of the active surface.

In an aspect, the cover comprises a channel to accommodate a cable from an inserted sensor, wherein the cover, preferably in the region of the channel, comprises at least one region with increased flexibility. The flexible region in the channel reduces force or torque that the cable exerts on the cover. The cover stays tightly seated on a surface even when the cable is moved.

The flexible area may comprise thinner or softer material or may be formed by a slit alongside the cable. Preferable two slits are formed alongside the cable.

In one aspect, the cover comprises a channel to accommodate a cable from an inserted sensor, the channel being open on the side of the opening of the chamber, the channel reaching through the sealing lip and into the chamber, the channel comprising channel walls perpendicular to the chamber opening and the sealing lip and a channel ceiling opposite of the opening and wherein the channel ceiling comprises slits along the inside of the chamber walls. The channel walls extend to the sealing lip, so that they provide sealing of the channel on a surface.

In an aspect, the cover is made of an elastic and/or flexible material, preferably silicone or a TPE like TPU, and/or wherein the material of the cover has a high absorption in the UV, visible, infrared and near-infrared spectrum. By this the cover is cost-efficient and easy to manufacture, for example using injection moulding.

In an aspect, the contact area of the sealing lip is sticky. A sticky surface may improve the light shielding and provides better seat on a surface.

Additionally or alternatively the contact area may comprise an adhesive that adheres the sealing lip to a surface. The adhesive may be applied partially or completely. The adhesive may be sufficient to fix the sensor and the cover, so that additional fixating means, like bandages, are not necessary.

The object is also solved by a sensor system comprising an optical sensor for measuring optical parameters in a scattering medium and a light shielding cover according to the invention, wherein the sensor is removably insertable into the chamber of the cover. A removable cover has the advantage that it could be applied only where it is needed and thus keeps the sensor simple and cheap. A removable cover is also cheaper and may be easier to clean or even could be sterilized. A removable cover could also be made disposable and for single use only.

The object is further solved by a sensor system comprising an optical sensor for measuring optical parameters in a scattering medium and a light shielding cover according to the invention, wherein the light shielding cover is integrally formed with the sensor. For some applications it may be beneficial that the cover is integrally formed with the sensor. The integral cover is always in the correct position, which may facilitate the application of the sensor system.

Preferred embodiments of the invention are described with reference to the attached figures C1 to C10. The shown embodiments are exemplary only and not limiting in any way.
- Fig. 1:: a perspective view from above of a light shielding cover according to an embodiment,
- Fig. 2:: a perspective view from below of a light shielding cover,
- Fig. 3: a side view of a long side of the cover of Fig. 1 with a cable channel,
- Fig. 4:: a side view of the opposing long side of Fig. 3,
- Fig. 5: a side view of a short side of the cover of Fig. 1,
- Fig. 6:: a side view of the opposing short side of Fig. 6,
- Fig. 7:: a top view of the cover of Fig. 1,
- Fig. 8:: a sectional view of an axial section of a sensor system with a cover of Fig. 1 and a sensor inserted in the cover,

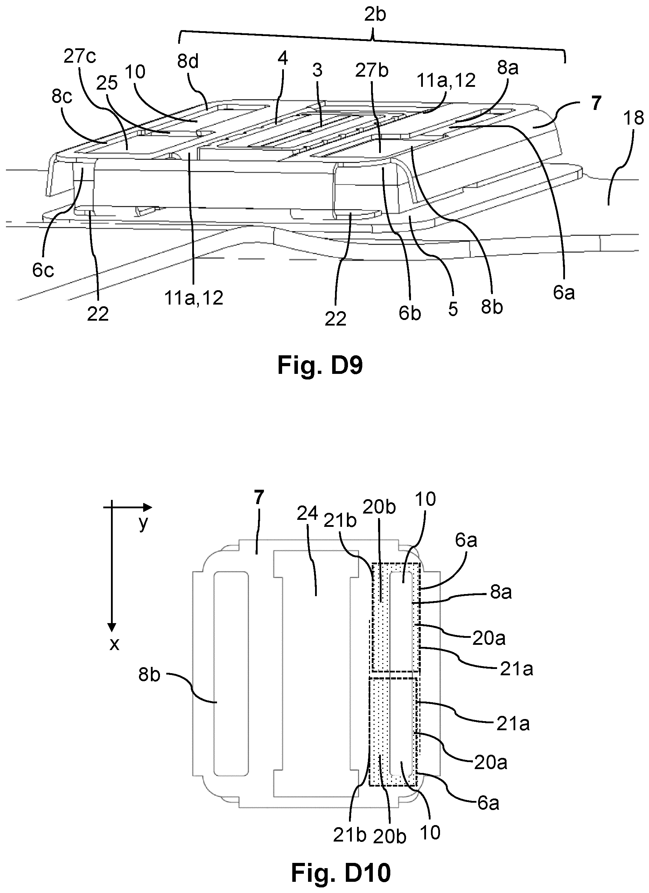

- Fig. 9:: a perspective view of Fig. 8,

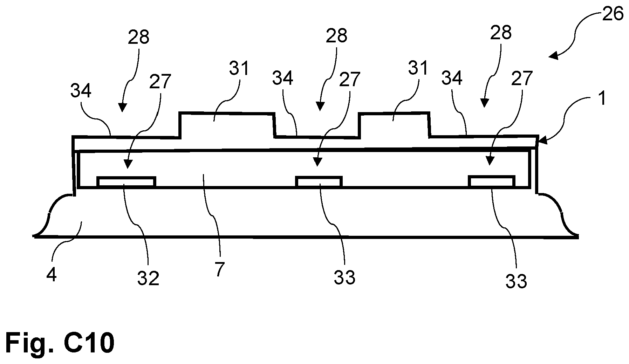

- Fig. 10:: a sectional view of a sensor system according to another embodiment.

Figures 1 to 7 show a light shielding cover 1 according to a first embodiment of the invention. Figures 8 and 9 show the cover 1 with a sensor 7 inserted in the cover 1. The first embodiment is described with respect to these figures 1 to 9 with no further reference to a specific figure.

The cover 1 comprises a chamber 2 for inserting an optical sensor 7, wherein the chamber 2 comprises an opening 3 through which the active surface 8 of an inserted optical sensor 7 is accessible. In the active surface 8 of the sensor 7 light sending and receiving means are placed. The opening 3 may also be used to insert the sensor 7 into the chamber 3.

The cover 1 further comprises a sealing lip 4 surrounding the chamber 2, wherein the sealing lip 4 protrudes from a rim 5 of the chamber 2 in a direction 9 normal to and away from the active surface 8 of an inserted sensor 7. The circumference of the sealing lip 4 at a contact area 6 is larger than the circumference of the chamber 2.

In this exemplary embodiment the chamber 2 is dimensioned to accommodate a rectangular sensor 7, with a long side (length) and a short side (width). The sensor and thus the chamber 2 are for example 4 cm long and 1 cm wide.

However different sensor can have differing sizes. The cover is preferably adapted to a specific size of a sensor 7 to be housed in the chamber 2. The invention is thus by no means limited to the mentioned dimensions.

The chamber 2 comprises a circumferential recessed rim 24 where a counterpart rim 25 of an optical sensor 7 bears on.

The sealing lip 4 comprises a bending area 10, where the sealing lip 4 is movable or deflectable relative to the chamber 2. For example, a deflection is enabled in the direction 9 normal to the active surface 8. The sealing lip 4 comprises a bending area 10 to enable such deflection or movement.

The bending area 10 is constituted of a first part 12, that extends from the rim 5 parallel to the plane 13 of the active surface 8 and a second part 14, that extends normal to the plane 16, wherein the first part 12 and second part 13 are connected by a circular, 90° bend 14.

Adjacent to the second part 14 of the bending area 10, the sealing lip 4 comprises a contact part or toe 15. The contact part 15 has a thicker wall than the bending area 10. The contact part 15 also comprises the contact area 6 on the inside of the sealing lip 4. The contact area 6 in the example is inclined to the plane 16. The inclination angle 36 here is around 40°. The inclination angle may be in the range of 20° to 60°.

Between the second part 14 and the contact part 15 a circumferential step 11 is formed resulting in an S-shaped cross section of the sealing lip. This also enables the sealing lip 4 to move like a gaiter.

Overall, the cover 1 has a concave form 35 that fits for example small heads or strong curved surfaces, see fig. 8.

The light shielding cover 1 comprises a channel 17 to accommodate a cable 18 from an inserted sensor 7. The channel 17 is open on the side of the opening 3 of the chamber 2. The channel reaches through the sealing lip 4 and into the chamber 2. This facilitates insertion of the sensor 7 with an attached cable 18 into the chamber 2.

The channel 18 comprises channel walls 19 that extend perpendicular to the chamber opening 2 and the sealing lip 4. The channel also comprises a channel ceiling 20 opposite of the opening and wherein the channel ceiling 20 comprises two slits 21 along the inside of the chamber walls 19. These slits 21 provide a region with increased flexibility. In use, when the cable 18 is moved, the slits enable the channel ceiling 20 to easily deflect.

Instead of the slits, that reach through the channel ceiling, only shallow trenches could be applied, so that thin membranes are formed in the trenches that allow deflection of the channel ceiling 20 as well while still closing off the channel.

In the shown example, the sealing lip 4 comprises a chamfer 22 in the direction of a long side of the chamber 2. The height of the sealing lip 4 is essentially zero at the base of the chamfer 22. Here the chamfer 22 results in the sealing lip being arranged asymmetrically around the chamber 2.

As could be seen in Fig. 5, the sealing lip 4 also comprises a chamfer 23 in a direction radial to the circumference of the chamber 2.

Fig. 8 and 9 show sensor systems 26 with a sensor 7 and a light shielding cover 1, wherein the sensor 7 is removably inserted in the cover 1. The sensor 7 in the example has three stiffer portions that comprise protrusions 27 where the sensor 7 is thicker than between these protrusions. In the region of these protrusions 27 or stiffer portions the light source and receivers are located.

The chamber 2 of the cover 1 here has a counterpart form of the sensor 7, so that the cover 1 tightly fits the sensor 7. The chamber 2 in the example comprises three relief portions 28 to accommodate these protrusions 27 of the optical sensor 7. In the shown example, these relief portions 28 are formed by openings 29 in the cover wall 30. However, the protrusions 27 of the sensor 7 do not project through the openings 29 to the outside of the chamber 2 but are recessed in the openings 29.

The cover wall 30 between these openings 28 form bars 31 that rest on the sensor surface. When fixing the sensor system 26 to a surface, for example to a head with a bandage, the bandage only contacts the cover wall 30 but not the protrusions 27 of the sensor 7. The force from the bandage is thus only applied to the bars 31. The bars 31 exert the force on the softer parts of the sensor but not to the stiffer parts or protrusions 27. The advantage here is that stiffer or rigid parts of the sensor, such as a light source or receiver is not pressed on the soft skin of for example a neonate.

Fig. 10 shows another embodiment of a sensor system 26. Here the sensor 7 also has a light source 32 and two receivers 33 spaced apart. The light source 32 and receiver 33 form stiffer portions 27 of the sensor.

Different from the embodiment shown in figures 1 to 9, the sensor 7 does not have protrusions in the stiffer portions 27 but has a flat surface. Hence the inner wall of the chamber is also flat to sit tightly on the sensor.

Nonetheless, the cover comprises three relief portions 28 that are formed by recesses 34 in the outside of the cover wall 30. Between the recesses 34 bars 31 are formed. In this embodiment force exerted from a bandage only is applied to the bars 31 that pass the force to the sensor outside the stiffer portions 27.

In Fig. 10 the recesses 34 could be on the inside of the channel wall or a combination of varying measures could be applied. Therefore, the invention is not limited to the shown examples.

In this embodiment the sealing lip 4 does not comprise a chamfer. Hence the sealing lip 4 has a uniform height and completely surrounds the chamber 2.

Apart from the two shown embodiments, there are numerous ways to form the relief portions. However important is that at the relief portions prevent force getting applied to stiffer portions of the sensor 7.

In yet another **aspect D)**, the disclosure concerns an optical sensor for medical applications, in particular designed for measuring physiological parameters in human or animal tissue. The sensor comprises an optical measurement arrangement with at least one light source arranged in a package or housing and at least one accompanying, preferably functionally connected to each other by a control circuit, optical detector. The at least one optical detector is intended and/or configured to receive light emitted by the at least one light source, after this measurement light has travelled through a portion of the tissue by random scattering processes.

The present disclosure further concerns a specific use of such an optical sensor.

Near-Infrared Spectroscopy (NIRS) is a special spectrophotometric method, which determines concentration of chromophores (for example oxy- and deoxyhemoglobin) in human tissue and, derived from these concentrations, tissue oxygen saturation (StO2). This is accomplished by illuminating the tissue at one or more spots with multispectral sources and measuring the received light intensity at one or more distant points. For this purpose, state-of-the-art oximeters use photodiodes as detectors and various LEDs with different emission wavelengths as light sources.

Optical NIRS sensors are nowadays widely used in clinical monitoring of patients. The accuracy and reliability of the optical measurements performed with such sensors in such applications is highly critical to the well-being of the patient.

It is therefore an object of the present invention to provide an optical sensor as introduced in the beginning which offers improved reliability and/or measurement accuracy. An additional goal of the invention is to simplify the manufacturing and assembly of such a sensor.

In accordance with the present invention, an optical sensor is provided according to claim 1, which solves the afore-mentioned problems at least partially. In particular, the invention proposes an optical sensor as introduced at the beginning, which is characterized in that the optical measurement arrangement is at least partially covered by a light shielding featuring at least one detector aperture which limits angles, at least in one direction, at which an active area of the at least one optical detector can receive light. Furthermore, it is proposed that the light shielding is self-aligned to the package by a positioning means. Accordingly, the positioning means can be in a direct mechanical contact with the package housing the at least one light source.

As is well known in the art, a light path can be inverted simply by exchanging the position of light source and detector. In such a case, the light shielding may be self-aligned to a package or housing accommodating said at least one optical detector. In other words, in this alternative, which is considered technically equivalent to the invention, the optical sensor (as introduced in the beginning) may be characterized in that the optical measurement arrangement is at least partially covered by a light shielding featuring at least one source aperture which limits angles, at least in one direction, at which the at least one light source can emit light, and the light shielding is self-aligned to the at least one detector, in particular to a housing of said detector, by a positioning means.

The invention thus proposes to align the light shielding directly to the package of the at least one light source. This approach is different from performing such an alignment to structures formed on a PCB. In the latter case, which is often used in the prior art, the placement tolerances of the light sources on the PCB will affect the accuracy of the alignment between the light shielding and the light source. This problem is thus prevented by the invention.

Most preferably, the positioning means can be an active positioning means. Such an active positioning means can provide/produce a positioning force that results in an active alignment of the light shielding w.r.t. the at least one light source.

In other words, thanks to the (in particular active) positioning means, the light shielding can (in particular actively, e.g., by means of a spring action) self-align itself relative to the package accommodating the at least one light source into a final assembly position.

The technical advantage of this approach is that due to the self-alignment/self-aligning through self-positioning of the light shielding relative to the package, the respective position of the at least one detector aperture provided by the light shielding relative to the at least one light source (i.e., in particular, relative to a set of light sources) arranged in the package or at least to the package itself can be accurately and automatically controlled. This greatly simplifies the assembly of the apertures and the total optical measurement arrangement. In addition, the approach proposed by the invention reduces tolerances between the at least one light source - that can be very accurately positioned with respect to the package - and the effective active area of the optical detector or between the active area of the at least one detector and said at least one light source (depending on the design of the light shielding), such that the measurement accuracy is improved.

Another advantage is that the light shielding can be assembled after completion of a pre-assembly of the measurement arrangement on a printed circuit board; holding the light shielding in place during the pre-assembly is thus not necessary. This simplifies the assembly.

More importantly, such an approach is particularly helpful when the optical measurement arrangement is intended for an optical calibration measurement, in which optical coupling efficiencies relevant to optical measurements performed with the at least one detector, are determined (by the optical sensor itself). This is because in such calibration measurements, the effective source-detector-separation (SDS) can be relatively short, and hence any variation of the effective SDS should be minimized, such that relative measurement errors are kept as small as possible.

Preferably, the positioning means can be formed as an integral part of the light shielding.

As already stated, the positioning means may also provide an actuating/positioning force for the self-positioning and/or self-alignment of the light shielding relative to the package. The positioning means may thus not only be a simple alignment structure, but it may actively move the light shielding into a final self-aligned assembly position on the package.

The at least one detector aperture can preferably be formed as an integral part of the light shielding.

According to a preferred embodiment, the light shielding may feature two opposing detector apertures which are symmetrically arranged with respect to a center of said at least one light source. In such a design, the at least one light source (for example located in the center of the measurement arrangement) can be used in conjunction with at least two opposing optical detectors, located below the respective detector aperture, for performing a highly accurate optical calibration measurement.

Alternatively or additionally, in particular if the light shielding is self-aligned to the at least one optical detector, the light shielding may feature two opposing source apertures which are symmetrically arranged with respect to a center of said at least one optical detector. In such a design, the at least one optical detector (for example located in the center of the measurement arrangement) can be used in conjunction with at least two opposing light sources, located below the respective source aperture, for performing a highly accurate optical calibration measurement.

The package or housing accommodating the at least one light source / a set of light sources can provide an optical shielding which prevents direct optical crosstalk between the at least one light source / the light sources and the at least one optical detector. In other words, the package can form an optical barrier between the light sources and the respective optical detector. Alternatively or additionally, such an optical barrier can be formed by the light shielding. The package may be, in particular, a ceramic package, which can provide excellent optical barrier properties. A combination of both is also possible.

The detector aperture of the light shielding may cover at least part of the active area (i.e., the area of the detector that is sensitive to light) of the at least one optical detector and thus define a sub-area of the active area that can receive light. It is to be understood that the source aperture can be defined within the package housing the light sources, for example. In this case, the relative position of the at least one source aperture to the at least one detector aperture will be defined as soon as the light shielding is self-aligned to the package. The use of a separate source aperture can be highly beneficial because in this case, some misplacement of the respective light source within the package may be tolerated as long as the light source is not covered too much. Depending on the application, some loss of emitted light, due to the coverage, may be acceptable, because the benefit of the source aperture is the improved measurement accuracy.

Alternatively, the source aperture may be integral to the light shielding (the light shielding may thus form the source aperture). This makes the sensor easier to assemble and saves costs.

The optical measurement arrangement, i.e., the at least one light source, in particular said set of light sources, arranged in the package and the at least one optical detector, can be mounted on a common printed circuit board (PCB), which may be designed as a flex-PCB. Thanks to the invention, placement tolerances of the package and the detector - within a certain scope - are rendered irrelevant since the detector aperture will be automatically positioned relative to the package and thus the SDS will be defined mainly by tolerances of the light shielding.

Each of the light sources arranged in the package can emit light around a respective characteristic center wavelength. The package can provide, for example, multiple different measurement wavelengths which are emitted by one of the respective light sources.

The at least one optical detector may comprise multiple receivers that each may be sensitive to all wavelengths or to selected wavelength ranges (e.g. by using appropriate optical filters, which may cover all or parts of the active area of the respective optical detector).

Both the at least one light source and the at least one optical detector of the measurement arrangement can be arranged such that they emit/receive light through a lower contact surface of the optical sensor that is brought into skin contact during an optical measurement.

The optical sensor presented so far can be further elaborated and implemented in various ways, which is described in the sub-claims and in the following:
For example, one embodiment suggests that the positioning means provide(s) a clamping force which clamps the light shielding, preferably onto the package and/or onto the at least one optical detector (in particular onto a package/housing accommodating said at least one detector).

For example, the positioning means may comprise (in particular it may be formed by) at least one spring element. In this case, it is preferred that the at least one spring element is integrally formed as part of the light shielding. The respective spring element can be formed as an elastic lip or an elastic arm, for example. An opposing force, interacting with a positioning force produced by said spring element, may be provided by another portion of the light shielding (for example a delimiter element), and this portion does not necessarily have to form a spring element.

According to a preferred embodiment, the light shielding features two opposing spring elements, for example in the form of two opposing elastically deformable lips or two opposing elastically deformable arms. Moreover, in a final aligned position of the light shielding (= final assembly position), the two opposing spring elements may clamp the package housing the at least one light source and/or the at least one optical detector (in particular a package/housing accommodating said at least one detector) from two opposing sides.

According to another embodiment, which may also be combined with the embodiment featuring two opposing spring elements, the light shielding may feature one spring element and one opposing delimiter element. The latter may provide an opposing force, interacting with the positioning force produced by said spring element. In such a case, these two opposing elements (spring element and opposing delimiter element) may clamp the package from two opposing sides.

For more accurately defining the position of the at least one light source and thus the location of emission of photons from the at least one light source, at least one source aperture may be provided as part of the measurement arrangement which limits angles, at least in one direction, at which the respective light source arranged in the package can emit light. This at least one source aperture may be part of the light shielding or a separate component of the measurement arrangement, in particular integrated into the package holding the at least one ligh source. Advantageously, in such a case, at least one effective source-detector-separation (SDS) between the at least one source aperture and the at least one detector aperture, in particular a respective detector aperture offered by the light shielding, may be defined by the self-aligned light shielding. This approach guarantees a highly precise definition of the SDS, which is important for optical calibration measurements.

We note here that the respective distance between the respective light source(s) and the optical detector(s) varies depending on the placement accuracy of these optoelectronic components on the PCB on which they are mounted. However, due to the use of the self-aligning light shielding, the distance between the respective source aperture(s) and detector aperture(s) (which ultimately defines the average distance that photons will travel from the respective light source to the respective detector and thus the effective SDS) will be unaffected by variations of the placement of the components on the PCB, as long as the detector aperture does not lie outside of the active area in direction of the relevant SDS.

Preferably, the respective detector aperture provided by the light shielding covers a distance in a first direction on the active area of the at least one optical detector that is significantly larger (e.g., 2x or 5x larger) than the placement accuracy of that detector along that first direction. As a result, a variation of the position of the optical detector along the longitudinal axis within the placement accuracy will not change the effective SDS. This is particularly helpful, when using a self-aligning optical shielding as proposed herein in a linear measurement arrangement, in which all optoelectronic components of the arrangement are arranged on a common longitudinal axis; in such a case, only variations of the placement of the components along this longitudinal axis (i.e., in the direction of the relevant SDS) must be compensated for an accurate measurement and this can be effectively achieved with the invention.

The at least one detector aperture provided by the light shielding may reduce an angular range of incoming light rays that the active area of the optical detector located beneath the respective detector aperture can detect. On the other hand, the aforementioned at least one source aperture (which may be part of the light shielding) may reduce an angular range of outgoing light rays that the respective light source, located beneath the respective source aperture, can emit.

Furthermore, the at least one detector aperture may cover two opposing sub-areas, respectively, located at opposing edges of an active area of the respective optical detector located below the respective detector aperture. In this case, the light shielding will cover two opposing edges and adjacent parts of the active area, in particular such that only a central portion of the active area can receive light. This design has the advantage that the width of the active area (in direction from edge to opposing edge) is independent of the placement accuracy. In case the at least one light source offers an extended active area, from which light is emitted, the respective source aperture may cover two opposing edges and adjacent parts of the active area of the light source, in particular such that only a central portion of the active area can emit light.

The angular range at which the active area of the optical detector can detect rays may be understood here as the field of view of the respective detector. Hence each detector aperture, in particular each respective portion of the respective detector aperture, of the light shielding can limit a respective field of view of a detector located below that aperture / aperture portion.

The intentional covering of sub-areas/portions of the active area by the optical shielding thus reduces the active area of the detector that is usable during an optical measurement performed by the optical sensor. In other words, the optical shielding can show a significant overlap, at least in one direction (preferably along a longitudinal axis of the measurement arrangement as described in more detail below), with the active area of the respective optical detector. The benefit is that the size of the active area that can receive light and, in particular, its location relative to the source aperture or light source will remain constant even when the position of the detector below the aperture is not perfectly controlled, which is often the case due to placement tolerances when mounting/reflow-soldering the detector onto a PCB of the optical sensor. A misplacement of the respective chip inside a chip housing will thus be less relevant (at least in the longitudinal direction) since the optical shielding determines the exact place and size of the active (sub-)area of the respective chip that can receive light, regardless of tolerances in the placement of the chip inside the housing.

The at least one source aperture mentioned before may be arranged inside of the package; in particular, it may be formed by the package, which houses the at least one light source. Alternatively, the at least one source aperture may be integrally formed as part of the light shielding.

Moreover, the light shielding may feature a window in which the package (holding the at least one light source) is arranged and through which the at least one light source (housed in the package) can emit light, in particular after the light has passed a respective source aperture. Alternatively, the at least one optical detector may be arranged in said window. In this case, the at least one detector will receive the light through said window.

According to a preferred embodiment, said window may be delimited on two opposing sides by a (respective) spring element, respectively. These two spring elements can thus form the positioning means (aligning the light shielding to the at least one light source or the at least one optical detector).

Another embodiment suggests that the light shielding forms the at least one source aperture (in particular as an integral part of the light shielding) and/or covers at least part of the light sources.

In addition, the light shielding may provide an optical barrier preventing direct optical crosstalk between the at least one light source and the at least one optical detector. Preferably, said optical barrier can be formed as an integral part of the light shielding, for example as a bent flap. The flap may even be soldered to a PCB on which the optical measurement arrangement is mounted. Thereby, tunnelling of light below the flap is effectively prevented.

The at least one source aperture can be formed as a slit aperture, in particular running along a transversal axis. This has the advantage that all light sources of the package can effectively have the same longitudinal coordinate.

Likewise, (in particular each of) the at least one detector aperture provided by the light shielding can be formed as a slit aperture, and this slit aperture may be running along said mentioned transversal axis.

Hence, the slit apertures forming the source aperture and each of the respective one of the at least one detector aperture may be arranged in parallel, respectively, i.e., they may be oriented along the same transversal axis.

One advantageous embodiment proposes that the light shielding offers two detector apertures each designed as a slit aperture and differing (from each other) in their respective slit width. These two detector apertures may be adjoint or separate from each other. In such a case, it is preferable if the two detector apertures are aligned along a common transversal axis.

Moreover, it is advantageous if the two detector apertures cover active areas, respectively, of two separate optical detectors arranged in a common housing. Such a design allows to fine-tune and optimize the respective active area of the respective optical detector arranged in the common housing that can receive light, which is highly beneficial for optimizing the sensitivity of the optical measurement performed with the respective detector. This is particularly true if the two separate optical detectors arranged in the common housing are configured to measure different measurement wavelengths (either by using appropriate optical filtering or time-multiplexing during the measurement), because in this case, each slit aperture can be optimized for use with one of the two different measurement wavelengths. For example, if one of the two different measurement wavelengths is more strongly absorbed in the tissue, this may be compensated by using a larger slit width of the corresponding detector aperture.

The two detector apertures each designed as a slit aperture can be separated by a thin separating bridge, which is blocking light; or the two slit areas of the two detector apertures may be merged into one open aperture. Seen from a different perspective, in such an embodiment the light shielding can offer a slit aperture featuring two neighbouring regions (along the transversal axis), which each region offering a different slit width. For example, the slit width may be smaller in a first region of the slit aperture than in a second region of the slit aperture. The slit width can be understood here as the width of the slit along a longitudinal axis, which is perpendicular to the transversal axis.

The individual widths of the slit apertures can thus vary depending on the optical detector underneath the respective slit aperture and depending on the field of view/amount of light required for that optical detector, which can depend on the source-detector-separation in which this particular optical detector is used in an optical measurement performed by the sensor. This approach is highly useful for maximizing the sensitivity of the individual optical measurements that can be performed with an individual optical detector of the sensor. For example, the longer the effective SDS and/or the stronger the respective measurement wavelength is absorbed in the tissue, the larger the slit width can be chosen to allow a meaningful compromise between spatial resolution (which is favoured by a slim slit aperture) and SNR (which is generally favoured by a broad slit aperture and hence a large active area that can receive photons).

In particular when the light shielding is self-aligned to the at least one optical detector, the light shielding may offer two source apertures, preferably each designed as a slit aperture. In such a case, it is preferable if the two source apertures are both running in the direction of a common transversal axis. For example, the two source apertures may be located to the left and to the right of the at least one optical detector.

It is to be noted here, that the light shielding can feature further optical apertures not designed as a slit. For example, when using an optical detector of the optical measurement arrangement for ambient light sensing, the light shielding can feature a corresponding rectangular aperture matching the full or a part of the active area of that detector used for ambient light sensing.

According to another preferred embodiment, the light shielding forms a Faraday shield which provides electromagnetic shielding for the at least one optical detector. Preferably, the light shielding may be formed as a bent sheet of metal, in particular with the at least one detector aperture and/or the at least one source aperture being cut out of the sheet; or, for example, from a plastic material featuring electrical conductors (e.g., in the form of embedded particles or in the form of a conductive coating applied to the plastic material) which render the plastic material electrically conductive. Such embodiments can all result in an effective Faraday shield. It is understood that the Faraday shield may be electrically grounded, in particular to a ground potential provided by a PCB on which the optical measurement arrangement is assembled.

Following the 2nd alternative of using a plastic material, the light shielding may be 3d-printed from a suitable polymer featuring electrically conductive particles or it may be injection moulded, to name just two possible approaches for fabrication. The two mentioned alternatives may also be combined: for example, the light shielding may be obtained from a bent and pre-cut metal shield, and injection moulded plastic pieces may be added for additional optical shielding, for example to form an additional optical barrier as mentioned above. A suitable metal coating that can enhance the electromagnetic-shielding properties but also simplify a soldering attachment of the optical shielding to a PCB is Zinc.

To greatly improve the robustness of the overall assembly and/or for providing electrical grounding of the light shielding (in particular its Faraday shield), one embodiment suggests that the light shielding features soldering feet which are soldered to a printed-circuit-board (PCB). This PCB may electrically contact the light sources and/or the at least one optical detector. Moreover, the soldering feet may be formed as bent flaps from a metal sheet forming the optical shieling. Each soldering feet may also offer a flat contact surface that can slide over a respective contact pad of the PCB during the self-alignment of the optical shielding. Finally, for simplifying the soldering attachment of the light shielding to the PCB, the light shielding can feature a soldering enhancing coating. The soldering feet can be designed as elastic elements which can provide a flexible mechanical contact to a contact pad of the PCB to which the light shielding is to be soldered.

According to another preferred embodiment, the flaps forming the soldering feet may each be bent from a respective sidewall of the optical shielding which covers a side facet of the at least one optical detector (and thus provide em-shielding to these side facets).

Preferably, a distance between two opposing soldering feet of the light shielding may be chosen such that there always remains at gap between the side facets of the at least one optical detector and the sidewalls of the optical shielding, taking placement tolerances of the at least one optical detector into account. Through this approach and as a result of the self-positioning/self-alignment of the light shielding provided by the active positioning means, the movement of the light shielding will be unimpeded by the at least one optical detector (no matter where exactly the optical detector is located within the placement accuracy).

The advantage of using soldering feet is that the solder connection of the light shielding to the PCB will define the z-height of the at least one detector aperture above the plane of the PCB. When the at least one optical detector is soldered onto the same PCB, the z-distance between the respective detector aperture and the active area at the top of the respective optical detector can thus be accurately controlled, which is important for accurately defining/limiting the field-of-view of that detector. Alternatively, the light shielding can be contacted to the PCB with contacting means such as conductive spring elements.

It is further noted that optical filters may be arranged on top of the respective active area of the respective optical detector comprised in the measurement arrangement. In this case, the respective optical filter (which may be implemented as a thin layer of silicone with embedded colorant with a specific optical absorption band, for example) will be located in between an inner side of the optical shielding and the active area of the respective detector.

According to one preferred embodiment, the optical measurement arrangement comprises at least two opposing optical detectors (preferably two opposing pairs of separate optical detectors arranged in a common housing, respectively) arranged on a longitudinal axis, and the package housing the at least one light source is also arranged on the longitudinal axis in between the two opposing optical detectors. In such an arrangement, it is beneficial if the light shielding features at least two opposing slit apertures, preferably with differing (i.e., different and/or varying) slit widths, which are positioned over the respective optical detector and thus define at least two (preferably differing) source-detector-separations along the longitudinal axis between the at least one light source and the respective optical detector. Such a linear optical measurement arrangement according to the invention is ideally suited for performing multiple optical calibration measurements rapidly and with high accuracy.

Another embodiment suggests that the package comprises at least four lights sources. These light sources may be aligned on a common transversal axis. At least two of the light sources, preferably all four light sources, may be emitting a different wavelength spectrum (e.g., centered about different center wavelengths), respectively. This design allows a multi-wavelength calibration measurement using the at least one optical detector of the arrangement.

Yet another design suggests that, to the left and to the right of the package along a longitudinal axis, two optical detectors may be arranged in a common housing, respectively, and that each active area of the respective optical detector is covered by a respective detector aperture, preferably in the form of a slit aperture. Here again, it may be of advantage depending on the measurement approach for improving the sensitivity of the measurement, if the slit widths of the detector apertures covering the detectors of two different housings differ from each other. Moreover, for the same reason it may be beneficial if at least one of the slit widths of the detector apertures covering the detectors arranged in the same housing differs from another one of the slit widths.

A highly preferred embodiment proposes that the sensor comprises two optical measurement arrangements (which may each be designed as detailed before), each comprising a respective at least one light source, in particular a respective set of light sources, arranged in a respective package, at least one accompanying optical detector, preferably and at least one source aperture. In such a sensor-design, each of the two measurement arrangements may be covered by a respective light shielding featuring at least one detector aperture and being self-aligned to the respective package, as has been previously described. Moreover, the two optical measurement arrangements can be arranged on a common longitudinal axis (to form a linear measurement arrangement) and each detector/source aperture may be designed as a slit aperture oriented along a transversal axis common to both optical measurement arrangements.

As already mentioned, each of the two measurement arrangements can have features as previously explained. The advantage of such a sensor layout is that each optical measurement arrangement can be used for performing optical calibration measurements characterizing the respective optical detector and/or light source used in the measurement and, afterwards, accurate optical measurements, taking the results of the calibration measurements into account, can be performed using (in particular alternately) a light source comprised in one (a first one) of the two optical measurement arrangements and an optical detector comprised in the other (second) optical measurement arrangement (and vice versa).

Finally, the invention suggests to use an optical sensor as described above and/or according to one of the claims directed towards an optical sensor in the following way for achieving the objective mentioned at the beginning: First, the sensor performs at least one optical calibration measurement to determine optical coupling efficiencies and/or correction factors using the at least one light source and the at least one optical detector comprised in the measurement arrangement of the optical sensor. We note that the at least one optical calibration measurement is based on at least one source-detector-separation defined by the optical shielding. It is evident, that the invention thus suggests that the optical sensor, in particular a controller of said optical sensor, may be configured to perform the steps just explained with respect to said use of the sensor.

In other words, the optical sensor first determines the coupling efficiencies and/or correction factors by performing an optical calibration measurement in tissue using the measurement arrangement and the accurate at least one SDS defined by the self-aligned optical shielding. Secondly, the optical sensor can then perform various optical measurements (typically ranging deeper into the tissue than the optical calibration measurement) using further light sources not comprised in the measurement arrangement covered by the light shielding but using a respective optical detector that is covered by a detector aperture provided by the light shielding. If the optical sensor does not move significantly (relative to the skin) during these different measurements, highly accurate measurements of the physiological parameters can be performed with the sensor.

Thus, in particular, a set of light sources housed in the package may be a first set and the optical sensor may feature at least one second set of light sources located on a longitudinal axis at a larger distance from the at least one optical detector (comprised in the measurement arrangement) than the first set. In this case, the optical sensor can measure physiological parameters in human tissue using the at least one second set and the at least one optical detector comprised in the measurement arrangement and considering the optical coupling efficiencies and/or correction factors determined in the optical calibration measurement.

This approach thus proposes to benefit from the accurate definition of relevant source-detector-separations provided by the light shielding according to the invention for performing highly accurate calibration measurements, which can then form the basis for following optical measurements of physiological parameters in human tissue, all using a sensor according to the invention.

Some examples of the present invention will now be described in more detail, although the present invention is not limited to these examples: for those skilled in the art, it is obvious that further examples of the present invention may be obtained by combining features of one or more of the patent claims with each other and/or with one or more features of an example as described or illustrated herein.

With reference to the accompanying Figures D1 to D13, where features with corresponding technical function are referenced with same numerals even when these features differ in shape or design:
- Fig. 1: is a perspective view of an optical sensor according to the invention, featuring three separate optical measurement arrangements,
- Fig. 2: shows a top view on the left outermost optical measurement arrangement of the sensor of Figure 1,
- Fig. 3: shows a top view on the optical measurement arrangement in the center of the sensor of Figure 1,
- Fig. 4: provides a schematic illustration of the lower contact side of the optical sensor of Figure 1,
- Fig. 5 and 6: show details of a light shielding according to the invention, which is part of the optical measurement arrangement of Figure 2,
- Fig. 7 and 8: show details of another light shielding according to the invention, which is part of the optical measurement arrangement of Figure 3,
- Fig. 9: provides a slightly inclined side view on the optical measurement arrangement of Figure 3,
- Fig. 10: provides a top view on the light shielding visible also in Figures 5 and 2 and illustrates two optical detectors positioned below the right detector aperture of the light shielding,

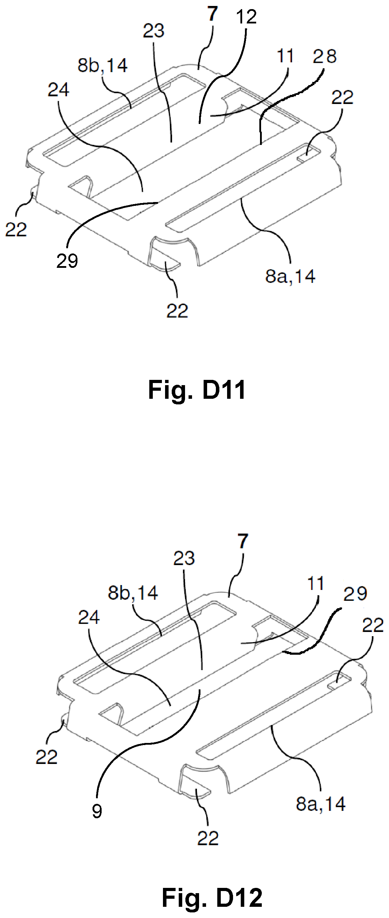

- Fig. 11: shows details of another light shielding according to the invention,
- Fig. 12: shows details of yet another light shielding according to the invention, and

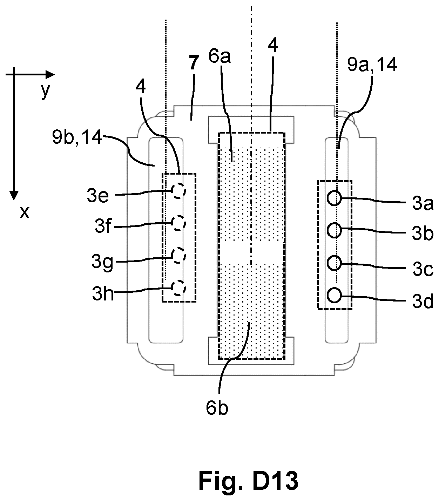

- Fig. 13: shows another possible embodiment of a light shielding 7 according to the invention.

Figure 1 shows an example of an optical sensor 1 according to the invention, which is designed as an optical near-infrared-spectroscopy (NIRS)-sensor. Such sensors are widely used in medical applications for live monitoring of blood oxygenation levels in the brain. In other words, the sensor 1 is capable of measuring blood oxygenation as a physiological parameter in e.g., human tissue, namely in deep lying brain layers.

As visible in Figure 4, the sensor 1 comprises three separate optical measurement arrangements 2a, 2b, 2c. Each optical measurement arrangement 2 features at least one light source 3 (namely multiple LEDs) and at least one accompanying optical detector 6, namely at least one respective photodiode. These optoelectronic components are mounted on and electrically contacted by a common printed circuit board (PCB) 18, which may be designed as a flex-PCB. Accordingly, the lower contact surface (here facing the reader) of the optical sensor 1, which is brought into skin contact during a measurement and through which light is emitted and received by the sensor 1, can be bent, such that the sensor 1 can be attached to the curved skull of a patient.

The left outermost optical measurement arrangement 2a, shown in more detail in Figure 2, features a total of four different LEDs (light sources 3a, 3b, 3c, 3d) forming a set of light sources 3, with each LED emitting a distinct and different wavelength ([λa1 .. λa4] - cf. Figure 4). The four LEDs are housed in a common ceramic package 4, which offers a slit aperture 14 (running along the transversal x-axis 16 of the sensor 1) that forms a source aperture 9. The source aperture 9 defines the exact location in y-direction (cf. Figure 1), at which light from the four LEDs can be emitted (along the z-axis - cf. Figure 1) into brain tissue, when the sensor 1 is placed on the skull of the patient. Moreover, the source aperture 9 limits angles in the y-direction, at which the respective LED arranged in the package 4 can emit light into the tissue. In the example of Figure 2, the source aperture 9 is arranged inside the package 4; however, a technically equivalent implementation would be a separate source aperture 9 that covers the package 4. Indeed, the source aperture 9 may be formed by the light shielding 7.

As visible in Figure 2, the optical measurement arrangement 2a is covered by a light shielding 7 that is formed as a bent metal sheet. As the light shielding 7 is thus electrically conductive and soldered to the PCB 18 using the illustrated soldering feet 22, it can provide an efficient electromagnetic shielding for the optical detectors 6 comprised in the arrangement 2b. The soldering feet 22 are formed as bent flaps and as part of the metal sheet forming the optical shieling 7 and each offer a flat contact surface that can slide over a respective contact pad 5 of the PCB 18 during a self-alignment of the optical shielding 7.

The two dotted lines and the dot-dash-line in between make it easy to recognize in Figure 2 that the light shielding 7 features two opposing detector apertures 8a and 8b, which are symmetrically arranged with respect to a center of said at least one light source 3. In other words, the respective y-distances (cf. Fig. 1) between the respective edge (dotted lines) of the detector aperture 8a/8b and the center line (dot-dash-line), on which the light sources 3 accommodated in the package 4 are arranged, are the same. The symmetrical arrangement is also highlighted by two dotted lines and a dot-dash-line in Figure 4. In fact, both optical measurement arrangements 2a and 2b in Figure 4 (as well as in the example of Figure 1) show such a symmetric arrangement of two opposing detector apertures 8a, 8b. In the example of Figure 13, where the light shielding 7 is self-aligned to the package 4 holding the two centrally arranged optical detectors 6a and 6b, the light shielding 7 offers two opposing source apertures 9a and 9b, which are symmetrically arranged with respect to a center of said at least one detector 6a, and 6b (as visible from the two dotted lines and the dot-dash-line). Also note that the respective slit widths of the two source apertures 9a, 9b differ in Figure 13.

The light shielding 7 shown in Figure 2 features two detector apertures 8a, 8b in the form of slit apertures 14a, 14b with a constant respective slit width 17a/17b (cf. Figure 5). Each of these apertures 8a, 8b can thus limit angles in the y-direction, at which an active area 10 of the respective detector 6 (covered by the light shielding 7) can receive light. This is important for performing meaningful NIRS-measurements, which requires accurately defined effective source-detector-separations (SDS) 13.

As visible in Figure 2 (and in Figures 5 and 6), the slit apertures 14a, 14b are also running along the transversal x-direction 16 of the optical sensor 1, similar to the source aperture 9. In Figure 5, it is notable that the respective slit widths 17a, 17b of the right aperture 8a and the left aperture 8b differ from each other. Such a design can optimize the relative sensitivity of the optical measurements performed at different wavelengths, because different wavelength dependent optical attenuations and differing quantum efficiencies of the detectors 6 for different wavelengths can be considered. In addition, the detectors may be used for different purposes and for light coming from different directions (ambient light, light for calibration, light for the actual measurement etc.). Accordingly, the aperture may have different shapes and may or may not be adjoint. One of the apertures may e.g. be a pin hole, while others form a slit of variable width.

The light shielding 7 of Figure 2 also comprises an active positioning means 11 in the form of two opposing spring elements 12a, 12b. As can be seen, the package 4 holding the set of light sources 3 is placed in a window 24 of the light shielding 7 (cf. Figure 6). Accordingly, the spring elements 12a, 12b, which are integral parts of the light shielding 7 and formed as elastically deformable lips, lie flat against the vertical sidewalls of the package 4. As each spring element 12a, 12b is slightly deformed, each spring element 12a, 12b provides a restoring force that acts as an actuating force 26 which actively aligns the light shielding 7 to the package 7. In other words, thanks to the active positioning means 11, the light shielding 7 can self-align to the package 4. The positioning means 11 also provide a clamping force which holds the light shielding 7 in place, relative to the package 4.

An alternative is shown in figure 11, where one spring element 11 and an opposing delimiter element 28 align the light shielding 7.

As a result, the relative positions/distances of the two detector apertures 8a, 8b of the light shielding 7 are accurately defined w.r.t. the source aperture 9. This relative positioning is important, because the relative locations of the detector apertures 8a, 8b w.r.t. the source aperture 9 define respective effective source-detector-separations (SDS) 13, which are illustrated in Figure 4 by dashed arrows. Accurate control of the respective SDS 13 is important for performing accurate optical calibration measurements with the optical measurement arrangement 2a.

In addition, placement inaccuracies of the respective optical detector 6 beneath the shielding 7, as a result of an assembly process on the PCB 18 (e.g., mounting of the photodiodes by reflow soldering), do not affect the resulting SDS 13. This may be understood with a look onto Figure 10, which illustrates two photodiodes (dashed boxes) as optical detectors 6a, 6b located on the PCB 18 and below the light shielding 7. As can be seen, the respective detector aperture 8a, 8b covers two opposing sub-areas 20a, 20b, respectively, which are located at opposing edges 21a, 21b of the active area 10 of the respective optical detector 6a, 6b which is located below the respective detector aperture 8a, 8b. Hence, some misalignment of the respective photodiode in the y-direction can be tolerated and this will not affect the size of the part of the active area 10, which can receive photons through the respective detector aperture 8a/8b, and, even more important, its relative location w.r.t. to the source aperture 9 (cf. Figure 2).

As may be understood from the perspective view of Figure 9, the respective detector aperture 8 formed by the light shielding 7 will reduce the angular range of incoming light rays that the respective active area 10 of the optical detector 6 beneath the respective detector aperture 8 can detect. This angular range is directly related to the slit width 17 of the respective aperture 8.

According to the design of the light shielding of Figure 2, the two opposing optical detectors 6a, 6d for example, which are arranged on the longitudinal y-axis 15, are approximately symmetrically arranged w.r.t. the light sources 3 arranged in the package 4; however, as the slit widths 17a, 17b and locations of the slit apertures 14a, 14b vary, two different source-detector-separations 13a, 13b are specified by the light shielding 7, as illustrated by the dashes arrows in Figure 2.

With reference to Figure 4, the invention thus suggests that the sensor 1 performs multiple optical calibration measurements to determine (for multiple wavelengths) respective optical coupling efficiencies and/or correction factors using the light sources 3 and the optical detectors 6 comprised in the optical measurement arrangement 2a and/or 2b. In these optical calibration measurements, several source-detector-separation (SDS) 13 as defined by the respective optical shielding 7 are employed (i.e., the respective values for the SDS as specified by the respective shielding 7 are considered during computing of the optical coupling efficiencies and/or correction factors). As a result, these optical calibration measurements will be highly accurate, because the respective SDS 13 is accurately defined by the light shielding 7 and basically independent of placement tolerances of the optical detectors 7 or the package 4 on the PCB 18. Note that the SDS does not need to be measured in direction of the y-axes and may in fact run under an angle thereto (cf. Figure 4).

As can be seen in Figure 1 and 4, the optical sensor 1 not only features two first sets of light sources 3 (namely the light sources 3 comprised in the optical measurement arrangement 2a and 2b) but also a second set of light sources 3 (providing a total of eight different measurement wavelength [λm1 .. λm8]) as part of the right outermost optical measurement arrangement 2c. All these light sources 3 are arranged on the longitudinal axis 15 of the optical sensor 1 (cf. Figure 1). However, the light sources 3 of the arrangement 2c are located at a larger distance from the optical detectors 6 of the arrangement 2b than the first set of light sources 3 of the arrangement 2b. The same is true with respect to the optical detectors 6 of the arrangement 2a and its set of light sources 3. As can be seen in Figure 4, optical measurements with large SDS 13 can thus be performed using the light sources 3 of the right outermost arrangement 2c (i.e., with said second set of light sources 3) and using optical detectors 6 of the arrangements 2a and/or 2b. We note at this point, that a large SDS 13 is tantamount to a large penetration depth of the measurement wavelength λₘᵢ into the tissue, i.e., such measurements are suitable for optically probing deep brain layers.

As a result, the optical sensor 1 can thus accurately measure the blood oxygenation as a physiological parameter in human tissue using the described second set of light sources 3 of the arrangement 2c and using the optical detectors 6 comprised in the measurement arrangements 2a and 2b. In these optical measurement, the optical coupling efficiencies and/or correction factors (determined in the precedent optical calibration measurements) are considered, which greatly improves the measurement accuracy. The use of the optical shielding 7 according to the invention thus results in a higher accuracy of the optical calibration measurements and thereby, ultimately, also in a higher accuracy of the performed optical measurements of said physiological parameter. In total, the accuracy of the oxygen saturation measurements is thus improved, to the benefit of the patient.

Figure 3 shows another embodiment of a light shielding 7 according to the invention, which covers the center optical measurement arrangement 2b that was already shown in Figure 1. It is notable, that the shielding 7 offers four slit apertures 8a, 8b, 8c, 8d which cover the respective active areas 10 of four different optical detectors 6a, 6b, 6c, 6d, each in the form of a respective photodiode. Seen from another perspective, the slit widths 17a and 17d of the detector apertures 8a and 8d covering the detectors 6a and 6d, which are located in two different separate housings 19a, 19b (cf. Figure 3) differ from each other, as visible in Figure 7. In addition, the slit widths 17a and 17b of the detector apertures 8a and 8b covering the detectors 6a and 6b which are arranged in the same housing 19a also differ from each other. As a result, a total of four different regions of variable size are defined w.r.t. the respective active region 10 of the respective optical detector 6 by the light shielding 7. Hence, it is possible to measure four different wavelengths with optimized sensitivity using the four detector apertures 8a, 8b, 8c, and 8d. As there are a two optical measurement arrangements 2a, 2b, for each of the eight measurement wavelengths a corresponding optical calibration measurement can be performed, using the auxiliary wavelength λai.

In summary, an optical sensor 1 is proposed which features a particular optical measurement arrangement 2 comprising at least one light source 3 and at least one accompanying optical detector 6. The arrangement 2 is distinguished in that it comprises a shielding 7 for shielding light from the at least one optical detector 6. The shielding 7 possesses an active positioning means 11 that can actively position the shielding 7 relative to the at least one light source 3. Thereby at least one, preferably several, source-detector-separation(s) 13 between the at least one light source 3 and the at least one optical detector 6 of the arrangement 2 can be accurately defined, even when the respective light source 3 and/or the respective detector 6 is/are slightly misplaced within the arrangement 2 due to placement tolerances. This approach thus delivers an automatic definition of important optical properties of the arrangement 2, which are relevant to calibration measurements performed with the arrangement 2.

Figures 11 and 12 show each a respective embodiment of a light shielding 7 according to the invention: In the example of Figure 11, the light shielding 7 features one spring element 12 that forms an active positioning means 11 that can produce a positioning force, and a delimiter element 28 (formed as an edge 29) opposing said spring element 12. In the final assembly position of the light shielding 7 (not shown in the figure), both the delimiter element 28 and the spring element 12 rest against the package 4 accommodating the at least one light source 3 of the optical measurement arrangement 2 and these two elements 12, 28 clamp the package 4 from two opposing sides.

In the example of Figure 12, the light shielding 7 features an edge 29 that forms a source aperture 9 for the light source 3 (not shown in the Figure) of the optical measurement arrangement 2 that is covered by the light shielding 7. In this embodiment, this edge 29 does not rest against the package 4 accommodating the at least one light source 3 but instead covers (partly) the at least one light source 3 / said package 4. In other words, the embodiment of Figure 12 is an example of a source aperture 9 that is formed as an integral part of the light shielding 7.

Figure 13 shows an embodiment of a light shielding 7 according to the invention, in which the light shielding features a source aperture 9a (limiting angles, at least in one direction, at which the respective light source 3a / 3b / 3c / 3d can emit light into the tissue), which is self-aligned to a package / housing 4 accommodating two optical detectors 6a, 6b. In this embodiment, the position of said package 4 / said detectors 6a, 6b will thus define the final position of the light shielding 7. The detectors 6a, 6b and the four light sources 3a - 3d (as well as the further four light sources 3e - 3h) may be part of an optical measurement arrangement 2 as detailed before. In this embodiment, placement tolerances of the respective light source 3 shown in Figure 13 will not affect the effective SDS between the respective light source 3 and the respective detector 6. This is because the respective source aperture 9a, 9b is aligned to both detectors 6a, 6b and the locations where the respective light source 3 can emit light into the tissue is thus defined by the light shielding 7, not by the position of the light source 3 (as long as the placement tolerance is not so large that the light source is completely blocked by the light shielding 7; note for example that the left light sources 3e - 3f are partly blocked by the light shielding 7, which will lead to a certain loss of light but can still result in a reasonable measurement of high accuracy).

Compared to the other embodiments, the light path has been inverted in the example of Figure 13 by exchanging the position of the at least one optical detector 6 and the at least one light source 3; however the light shielding 7 could also be aligned to the housing/package 4 accommodating the at least one optical detector 6 without exchanging the positions of light source and detector: for example, when aligning the light shielding 7 to the detectors 6 shown in Figure 2, the source aperture 9 would then be positioned above the light sources 3 in the center of the arrangement 2.

### List of reference numerals

**With respect to Figures B1 to B7**
- 1: sensor
- 2: sensor head (located at the distal end of 1)
- 3: cable (for routing electricals signal from and to 2)
- 4: contact plane (defined by 2 and to be brought in contact with tissue during a measurement)
- 5: light source
- 6: detector
- 7: carrier substrate (carrying 5 and 6)
- 8: flexible printed circuit board (flex-PCB)
- 9: lower member (of 2)
- 10: middle portion (of 8/27)
- 11: pillar (for supporting 8)
- 12: contact surface (of 2 defining 4)
- 13: window (in 11 for transmitting light emitted by 5 or received by 6)
- 14: supporting surface
- 15: supporting member (for supporting 7)
- 16: longitudinal y-axis
- 17: transverse x-axis
- 18: z-axis
- 19: metal housing
- 20: S-curve
- 21: inflection point (of 20 / 8)
- 22: center area (of 12)
- 23: light emission
- 24: light reception
- 25: proximal end (of 7)
- 26: interspace (between 11)
- 27: signal carrier (either an electrical conductor or a light guide, in particular a waveguide)
- 28: electrical conductor
- 29: light guide
- 30: rib (running in y-direction and serving as an optical barrier for mitigating optical cross-talk between 5 and 6)
- 31: proximal portion (of 8/27)
- 32: distal portion (of 8/27)
- 33: supporting structure
- 34: lower surface (of 3)

**With respect to Figures C1 to C10**
- 1: light shielding cover
- 2: chamber
- 3: opening
- 4: sealing lip
- 5: rim of chamber
- 6: contact area of sealing lip
- 7: sensor
- 8: active surface of sensor
- 9: direction normal to active surface of sensor
- 10: bending area
- 11: step
- 12: first part
- 13: second part
- 14: bend
- 15: contact part
- 16: plane of active surface
- 17: channel
- 18: cable
- 19: channel wall
- 20: channel ceiling
- 21: slit
- 22: chamfer in circumferential direction
- 23: chamfer normal to circumferential direction
- 24: rim in chamber
- 25: rim at sensor
- 26: sensor system
- 27: stiffer portion/protrusion
- 28: relief portion
- 29: opening
- 30: cover wall
- 31: bar
- 32: light source
- 33: receiver
- 34: recess
- 35: concave form
- 36: inclination angle

**With respect to Figures D1 to D13**
- 1: optical sensor
- 2: optical measurement arrangement
- 3: light source (e.g., a LED or a laser diode)
- 4: package (housing several 3 or one / several 6)
- 5: contact pad (of 18)
- 6: optical detector (e.g., a photodiode)
- 7: light shielding
- 8: detector aperture
- 9: source aperture
- 10: active area (of 6)
- 11: positioning means
- 12: spring element
- 13: source-detector-separation (SDS)
- 14: slit aperture
- 15: longitudinal axis
- 16: transversal axis
- 17: slit width (of 14)
- 18: printed-circuit-board
- 19: housing (for 6)
- 20: sub-area (of 10)
- 21: edge (of 10)
- 22: soldering feet
- 23: optical barrier
- 24: window (for receiving 4 or 6)
- 25: separation (between 6)
- 26: actuating force (provided by 11/12)
- 27: optical filter layer (e.g., provided in the form of a thin silicone film comprising absorbents than can block certain wavelengths to be suppressed)
- 28: delimiter element
- 29: edge

## Claims

1. **Sensor system (26)** comprising
- an optical sensor (7) designed for measuring optical parameters in a scattering medium and
- a light shielding cover (1),
- wherein an active surface (8) of the optical sensor (7) is to be placed on a surface of the medium during measuring, and wherein the cover (1) comprises:
- a chamber (2) for inserting the optical sensor (7), wherein the chamber (2) comprises an opening (3) through which the active surface (8) of the inserted optical sensor (7) is accessible, and
- a sealing lip (4) surrounding the chamber (2),
- wherein the sealing lip (4) protrudes from the chamber (2) in a direction normal to and away from the active surface (8) of the inserted sensor (7), and
- wherein a circumference of the sealing lip (4) at a contact area (6) is larger than a circumference of the chamber (2) and
- wherein the sealing lip (4) has an S-shaped cross section and comprises a bending area (10), such that the sealing lip (4) can deflect relative to the chamber (2) and adapt to different curvatures of heads for providing tight shielding from ambient light, and
- at least one bend of the S-shape constitutes the bending area (10), which is thus formed in the form of a bellow.

2. Sensor system (26) according to claim 1,
- wherein the chamber (2) comprises an opening (3) through which the active surface (8) of the inserted optical sensor (7) is accessible, and
- wherein the chamber (2) comprises one or more relief portions (28) to accommodate protrusions (27) or stiffer portions (27) of the optical sensor (7),
- wherein one of the one or more relief portions (28) comprises a recess (34) in the inside and/or the outside of the chamber wall (30) at the location of the respective protrusion (27) or stiffer portion (27) of the optical sensor (7).

3. Sensor system (26) according to claim 2, wherein the chamber (2) has a counterpart form of the optical sensor (7) to be housed and/or
- wherein the recess (34) of the one or more relief portions (28) comprises an opening (29) in the chamber wall (30).

4. Sensor system (26) according to any one of the preceding claims, wherein the sealing lip (4) comprises a circumferential step (11).

5. Sensor system (26) according to any one of the preceding claims, wherein the cover (1) has a concave cross section, at least in an axial direction of the cover (1).

6. Sensor system (26) according to any one of the preceding claims, wherein the contact area (6) of the sealing lip (4) is inclined with respect to the plane (16) of the active surface (8), especially wherein the inclination angle (36) is between 20 and 60 degrees, preferably tapering in an axial direction of the cover (1).

7. Sensor system (26) according to any one of the preceding claims, wherein the sealing lip (4) comprises a chamfer (22) in a circumferential direction, especially wherein a height of the sealing lip (4) is essentially zero at the base of the chamfer (22).

8. Light shielding cover according to any one of the preceding claims, wherein the sealing lip (4) is asymmetrically arranged around the chamber (2).

9. Sensor system (26) according to any one of the preceding claims, wherein the chamber (2) comprises a circumferential recessed rim (24) where a counterpart rim (25) of an optical sensor (7) bear on.

10. Sensor system (26) according to any one of the preceding claims, wherein the cover (1) comprises a channel (17) to accommodate a cable (18) from an inserted sensor (7), wherein the cover (1), preferably in the region of the channel (17), comprises at least one region with increased flexibility, such as a slit (21) alongside the cable.

11. Sensor system (26) according to the previous claim, wherein the channel (17) is open on the side of the opening (3) of the chamber (3), the channel (17) reaching through the sealing lip (4) and into the chamber (2), the channel (17) comprising channel walls (19) perpendicular to the chamber (2) opening (3) and the sealing lip (4) and a channel ceiling (20) opposite of the opening (3) and wherein the channel ceiling (20) comprises slits (21) along the inside of the chamber walls (19).

12. Sensor system (26) according to any one of the preceding claims, wherein the cover (1) is made of an elastic material, preferably silicone or a TPE like TPU, and/or wherein the material of the cover has a high absorption in the UV, visible, infrared and near-infrared spectrum.

13. Sensor system (26) according to any one of the preceding claims, wherein the contact area (6) of the sealing lip (4) is sticky and/or comprises an adhesive.

14. Sensor system (26) according to claim 1, wherein the sensor (7) is removably insertable into the chamber (2) of the cover (1).

15. Sensor system (26) according to claim 1, wherein the light shielding cover (1) is integrally formed with the sensor (7).
